# EUROPEAN PATENT SPECIFICATION

(11) **EP 1 299 356 B1**
(45) Date of publication and mention of the grant of the patent: **11.02.2004**
(21) Application number: 01941407.7
(22) Date of filing: 14.06.2001
(51) Int. Cl.: C07D 211/46, C07D 211/58, C07D 211/22, C07D 207/12, C07D 295/08, C07D 295/10, A61K 31/295

(54) **NOVEL COMPOUNDS**
NEUE VERBINDUNGEN
NOUVEAUX COMPOSES

(30) Priority: 20.06.2000 SE 0002330; 31.10.2000 SE 0003980
(43) Date of publication of application: 09.04.2003
(73) Proprietor: AstraZeneca AB, 151 85 Södertälje (SE)
(72) Inventor: ERIKSSON, Tomas, S-221 87 Lund (SE); HENRIKSSON, Krister, S-221 87 Lund (SE)
(86) International application number: PCT/SE2001/001378
(87) International publication number: WO 2001/098272

(56) References cited:
- WO-A1-00/35449
- WO-A1-00/35451

## Description

The present invention relates to novel compounds, processes for their preparation, pharmaceutical compositions containing them and their use in therapy.

Chemokines play an important role in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. The chemokine superfamily can be divided into two main groups exhibiting characteristic structural motifs, the Cys-X-Cys (C-X-C) and Cys-Cys (C-C) families. These are distinguished on the basis of a single amino acid insertion between the NH-proximal pair of cysteine residues and sequence similarity.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils such as human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (MIP-1α and MIP-1β).

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10, CXCR1, CXCR2, CXCR3 and CXCR4. These receptors represent good targets for drug development since agents which modulate these receptors would be useful in the treatment of disorders and diseases such as those mentioned above.

In accordance with the present invention, there is therefore provided a compound of general formula 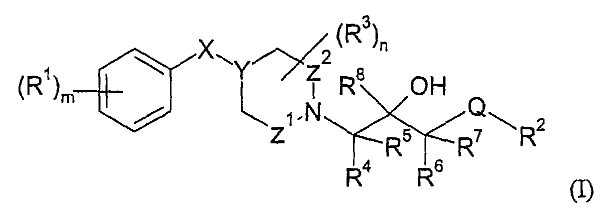 wherein:
mis0,1,2or3;
each R¹ independently represents halogen, cyano, nitro, carboxyl, hydroxyl, C₃-C₆ cycloalkyl, C₁-C₆ alkoxy, C₁-C₆ alkoxycarbonyl, C₁-C₆ haloalkyl, C₁-C₆ haloalkoxy, -NR⁹R¹⁰, C₃-C₆ cycloalkylamino, C₁-C₆ alkylthio, C₁-C₆ alkylcarbonyl, C₁-C₆ alkylcarbonylamino, sulphonamido (-SO₂NH₂), C₁-C₆ alkylsulphonyl, -C(O)NR¹¹R¹², -NR¹³C(O)-(NH)ₚR¹⁴, phenyl, or C₁-C₆ alkyl optionally substituted by carboxyl or C₁-C₆ alkoxycarbonyl;
p is 0 or 1;
X represents an oxygen atom or a CH₂, OCH₂, CH₂O, CH₂NH, NH, carbonyl or sulphonyl group and Y represents a nitrogen atom or a CH or C(OH) group, provided that when X represents an oxygen atom or a CH₂O, CH₂NH or NH group, then Y represents a CH group;
Z¹ represents a bond or a group (CH₂)_{q} where q is 1 or 2;
Z² represents a bond or a group CH₂, with the proviso that Z¹ and Z² do not both simultaneously represent a bond;
Q represents an oxygen or sulphur atom or a group CH₂ or NH;
R² represents a group 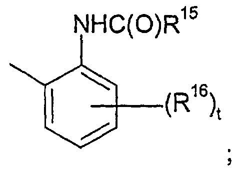
n is 0, 1 or 2;
each R³ independently represents a C₁-C₆ alkyl, C₁-C₆ alkoxycarbonyl, -CH₂OH or carboxyl group;
R⁴, R⁵, R⁶ and R⁷ each independently represent a hydrogen atom or a C₁-C₆ alkyl group, or R⁴, R⁵, R⁶ and R⁷ together represent a C₁-C₄ alkylene chain linking the two carbon atoms to which they are attached to form a 4- to 7-membered saturated carbocycle, or R⁵, R⁶ and R⁷ each represent a hydrogen atom and R⁴ and R⁸ together with the carbon atoms to which they are attached form a 5- to 6-membered saturated carbocycle;
R⁸ represents a hydrogen atom, a C₁-C₆ alkyl group or is linked to R⁴ as defined above;
R⁹ and R¹⁰ each independently represent a hydrogen atom or a C₁-C₆ alkyl group, or R⁹ and R¹⁰ together with the nitrogen atom to which they are attached form a 4- to 7-membered saturated heterocycle;
R¹¹ and R¹² each independently represent a hydrogen atom or a C₁-C₆ alkyl group optionally substituted by C₁-C₆ alkoxycarbonyl;
R¹³ represents a hydrogen atom or a C₁-C₆ alkyl group;
R¹⁴ represents a hydrogen atom, or a C₁-C₆ alkyl group optionally substituted by carboxyl, C₁-C₆ alkoxy or C₁-C₆ alkoxycarbonyl;
R¹⁵ represents a group C₂-C₆ alkyl, C₂-C₆ alkenyl, C₃-C₆ cycloalkyl, C₅-C₆ cycloalkenyl, adamantyl, phenyl or a saturated or unsaturated 5- to 10-membered heterocyclic ring system comprising at least one heteroatom selected from nitrogen, oxygen and sulphur, wherein each group may be optionally substituted by one or more substituents independently selected from nitro, hydroxyl, oxo, halogen, carboxyl, C₁-C₆ alkyl, C₁-C₆ alkoxy, C₁-C₆ alkylthio, C₁-C₆ alkylcarbonyl, C₁-C₆ alkoxycarbonyl, phenyl and -NHC(O)-R¹⁷, with the proviso that R¹⁵ does not represent an unsubstituted 1-pyrrolidinyl, an unsubstituted 1-piperidinyl or an unsubstituted 1-hexamethyleneiminyl (1-homopiperidinyl) group;
t is 0, 1, 2 or 3;
each R¹⁶ independently represents halogen, cyano, nitro, carboxyl, hydroxyl, C₃-C₆ cycloalkyl, C₁-C₆ alkoxy, C₁-C₆ alkoxycarbonyl, C₁-C₆ haloalkyl, C₁-C₆ haloalkoxy, -NR¹⁸R¹⁹, C₃-C₆ cycloalkylamino, C₁-C₆ alkylthio, C₁-C₆ alkylcarbonyl, C₁-C₆ alkylcarbonylamino, sulphonamido (-SO₂NH₂), C₁-C₆ alkylsulphonyl, -C(O)NR²⁰R²¹, -NR²²C(O)(NH)ᵥR²³, phenyl, or C₁-C₆ alkyl optionally substituted by carboxyl or C₁-C₆ alkoxycarbonyl;
R¹⁷ represents a C₁-C₆ alkyl, amino (-NH₂) or phenyl group;
R¹⁸ and R¹⁹ each independently represent a hydrogen atom or a C₁-C₆ alkyl group, or R¹⁸ and R¹⁹ together with the nitrogen atom to which they are attached form a 4- to 7-membered saturated heterocycle;
R²⁰ and R²¹ each independently represent a hydrogen atom or a C₁-C₆ alkyl group optionally substituted by C₁-C₆ alkoxycarbonyl;
v is 0 or 1;
R²² represents a hydrogen atom or a C₁-C₆ alkyl group; and
R²³ represents a hydrogen atom, or a C₁-C₆ alkyl group optionally substituted by carboxyl, C₁-C₆ alkoxy or C₁-C₆ alkoxycarbonyl;
or a pharmaceutically acceptable salt or solvate thereof.

In the context of the present specification, an alkyl or alkenyl substituent group or an alkyl or alkenyl moiety in a substituent group may be linear or branched. In the definition of R¹⁵, it should be noted that the unsaturated 5- to 10-membered heterocyclic ring system may be aliphatic or aromatic.

The integer m is preferably 1 or 2.

Each R¹ independently represents halogen (e.g. chlorine, fluorine, bromine or iodine), cyano, nitro, carboxyl, hydroxyl, C₃-C₆ cycloalkyl (cyclopropyl, cyclobutyl, cyclopentyl or cyclohexyl), C₁-C₆, preferably C₁-C₄, alkoxy (e.g. methoxy, ethoxy, n-propoxy or n-butoxy), C₁-C₆, preferably C₁-C₄, alkoxycarbonyl (e.g. methoxycarbonyl or ethoxycarbonyl), C₁-C₆, preferably C₁-C₄, haloalkyl (e.g. trifluoromethyl), C₁-C₆, preferably C₁-C₄, haloalkoxy (e.g. trifluoromethoxy), -NR⁹R¹⁰, C₃-C₆ cycloalkylamino (e.g. cyclopropylamino, cyclobutylamino, cyclopentylamino or cyclohexylamino), C₁-C₆, preferably C₁-C₄, alkylthio (e.g. methylthio or ethylthio), C₁-C₆, preferably C₁-C₄, alkylcarbonyl (e.g. methylcarbonyl, ethylcarbonyl, n-propylcarbonyl, isopropylcarbonyl, n-butylcarbonyl, n-pentylcarbonyl or n-hexylcarbonyl), C₁-C₆, preferably C₁-C₄, alkylcarbonylamino (e.g. methylcarbonylamino or ethylcarbonylamino), sulphonamido, C₁-C₆, preferably C₁-C₄, alkylsulphonyl (e.g. methylsulphonyl, ethylsulphonyl, n-propylsulphonyl, isopropylsulphonyl, n-butylsulphonyl, n-pentylsulphonyl or n-hexylsulphonyl), -C(O)NR¹¹R¹², -NR¹³C(O)-(NH)ₚR¹⁴, phenyl, or C₁-C₆, preferably C₁-C₄, alkyl (e.g. methyl, ethyl, n-propyl, isopropyl, n-butyl, isobutyl, tert-butyl, n-pentyl or n-hexyl) optionally substituted by carboxyl or C₁-C₆, preferably C₁-C₄, alkoxycarbonyl (e.g. methoxycarbonyl or ethoxycarbonyl).

Most preferably, each R¹ independently represents halogen (particularly chlorine or fluorine), cyano, nitro, C₁-C₆ alkoxy (especially methoxy), C₁-C₆ alkylcarbonyl (especially methylcarbonyl) or C₁-C₆ alkylcarbonylamino (particularly methylcarbonylamino). Each R¹ especially represents halogen or cyano.

Preferably X represents an oxygen atom or a CH₂ or NH group.

Preferred combinations of Y, Z¹ and Z² include:

| **Y** | **Z**^{**1**} | **Z**^{**2**} |
|---|---|---|
| CH | CH₂ | bond |
| CH | bond | CH₂ |
| CH | CH₂ | CH₂ |
| CH | (CH₂)₂ | bond |
| N | CH₂ | CH₂ |

Q preferably represents an oxygen atom.

Each R³ independently represents a C₁-C₆, preferably C₁-C₄, alkyl (e.g. methyl, ethyl, n-propyl, isopropyl, n-butyl, isobutyl, tert-butyl, n-pentyl or n-hexyl), C₁-C₆, preferably C₁-C₄, alkoxycarbonyl (e.g. methoxycarbonyl or ethoxycarbonyl), -CH₂OH or carboxyl group. It is preferred that R³ represents a methyl, methoxycarbonyl, ethoxycarbonyl, -CH₂OH or carboxyl group.

R⁴, R⁵, R⁶ and R⁷ each independently represent a hydrogen atom or a C₁-C₆, preferably C₁-C₄, alkyl (e.g. methyl, ethyl, n-propyl, isopropyl, n-butyl, isobutyl, tert-butyl, n-pentyl or n-hexyl), or R⁴, R⁵, R⁶ and R⁷ together represent a C₁-C₄ alkylene chain linking the two carbon atoms to which they are attached to form a 4- to 7-membered saturated carbocycle (e.g. cyclohexyl or preferably cyclopentyl), or R⁵, R⁶ and R⁷ each represent a hydrogen atom and R⁴ and R⁸ together with the carbon atoms to which they are attached form a 5- to 6-membered saturated carbocycle (preferably cyclopentyl).

R⁸ represents a hydrogen atom, a C₁-C₆, preferably C₁-C₄, alkyl group (e.g. methyl, ethyl, n-propyl, isopropyl, n-butyl, isobutyl, tert-butyl, n-pentyl or n-hexyl) or is linked to R⁴ as defined above.

R⁹ and R¹⁰ each independently represent a hydrogen atom or a C₁-C₆, preferably C₁-C₄, alkyl group (e.g. methyl, ethyl, n-propyl, isopropyl, n-butyl, isobutyl, tert-butyl, n-pentyl or n-hexyl), or R⁹ and R¹⁰ together with the nitrogen atom to which they are attached form a 4- to 7-membered saturated heterocycle.

R¹¹ and R¹² each independently represent a hydrogen atom or a C₁-C₆, preferably C₁-C₄, alkyl group (e.g. methyl, ethyl, n-propyl, isopropyl, n-butyl, isobutyl, tert-butyl, n-pentyl or n-hexyl) optionally substituted by a C₁-C₆, preferably C₁-C₄, alkoxycarbonyl substituent group.

R¹³ represents a hydrogen atom or a C₁-C₆, preferably C₁-C₄, alkyl group (e.g. methyl, ethyl, n-propyl, isopropyl, n-butyl, isobutyl, tert-butyl, n-pentyl or n-hexyl).

R¹⁴ represents a hydrogen atom, or a C₁-C₆, preferably C₁-C₄, alkyl group (e.g. methyl, ethyl, n-propyl, isopropyl, n-butyl, isobutyl, tert-butyl, n-pentyl or n-hexyl) optionally substituted by carboxyl, C₁-C₆, preferably C₁-C₄, alkoxy or C₁-C₆, preferably C₁-C₄, alkoxycarbonyl.

R¹⁵ represents a group C₂-C₆, preferably C₂-C₄, alkyl group (e.g. ethyl, n-propyl, isopropyl, n-butyl, isobutyl, tert-butyl or n-pentyl), C₂-C₆, preferably C₂-C₄, alkenyl, C₃-C₆ cycloalkyl (e.g. cyclobutyl or cyclopentyl), C₅-C₆ cycloalkenyl, adamantyl, phenyl or a saturated or unsaturated 5- to 10-membered heterocyclic ring system comprising at least one heteroatom selected from nitrogen, oxygen and sulphur, wherein each group may be optionally substituted by one or more (e.g. one, two, three or four) substituents independently selected from nitro, hydroxyl, oxo, halogen (e.g: fluorine, chlorine, bromine or iodine), carboxyl, C₁-C₆, preferably C₁-C₄, alkyl (e.g. methyl, ethyl, n-propyl, isopropyl, n-butyl, isobutyl, tert-butyl, n-pentyl or n-hexyl), C₁-C₆, preferably C₁-C₄, alkoxy (e.g. methoxy, ethoxy, n-propoxy or n-butoxy), C₁-C₆, preferably C₁-C₄, alkylthio (e.g. methylthio or ethylthio), C₁-C₆, preferably C₁-C₄, alkylcarbonyl (e.g. methylcarbonyl, ethylcarbonyl, n-propylcarbonyl, isopropylcarbonyl, n-butylcarbonyl, n-pentylcarbonyl or n-hexylcarbonyl), C₁-C₆, preferably C₁-C₄, alkoxycarbonyl (e.g. methoxycarbonyl or ethoxycarbonyl), phenyl and -NHC(O)-R¹⁷.

The saturated or unsaturated 5- to 10-membered heterocyclic ring system may be monocyclic or polycyclic (e.g. bicyclic) and may comprise up to four heteroatoms independently selected from nitrogen, oxygen and sulphur. Examples of ring systems that may be used include pyrrolidinyl, piperidinyl, pyrazolyl, thiazolidinyl, thienyl, isoxazolyl, thiadiazolyl, pyrrolyl, furanyl, thiazolyl, indolyl, quinolinyl, benzimidazolyl, triazolyl, tetrazolyl and pyridinyl.

Each R¹⁶ independently represents halogen (e.g. chlorine, fluorine, bromine or iodine), cyano, nitro, carboxyl, hydroxyl, C₃-C₆ cycloalkyl (cyclopropyl, cyclobutyl, cyclopentyl or cyclohexyl), C₁-C₆, preferably C₁-C₄, alkoxy (e.g. methoxy, ethoxy, n-propoxy or n-butoxy), C₁-C₆, preferably C₁-C₄, alkoxycarbonyl (e.g. methoxycarbonyl or ethoxycarbonyl), C₁-C₆, preferably C₁-C₄, haloalkyl (e.g. trifluoromethyl), C₁-C₆, preferably C₁-C₄, haloalkoxy (e.g. trifluoromethoxy), -NR¹⁸R¹⁹, C₃-C₆ cycloalkylamino (e.g. cyclopropylamino, cyclobutylamino, cyclopentylamino or cyclohexylamino), C₁-C₆, preferably C₁-C₄, alkylthio (e.g. methylthio or ethylthio), C₁-C₆, preferably C₁-C₄, alkylcarbonyl (e.g. methylcarbonyl, ethylcarbonyl, n-propylcarbonyl, isopropylcarbonyl, n-butylcarbonyl, n-pentylcarbonyl or n-hexylcarbonyl), C₁-C₆, preferably C₁-C₄, alkylcarbonylamino (e.g. methylcarbonylamino or ethylcarbonylamino), sulphonamido, C₁-C₆, preferably C₁-C₄, alkylsulphonyl (e.g. methylsulphonyl, ethylsulphonyl, n-propylsulphonyl, isopropylsulphonyl, n-butylsulphonyl, n-pentylsulphonyl or n-hexylsulphonyl), -C(O)NR²¹R²², -NR²³C(O)-(NH)ᵥR²⁴, phenyl, or C₁-C₆, preferably C₁-C₄, alkyl (e.g. methyl, ethyl, n-propyl, isopropyl, n-butyl, isobutyl, tert-butyl, n-pentyl or n-hexyl) optionally substituted by carboxyl or C₁-C₆, preferably C₁-C₄, alkoxycarbonyl (e.g. methoxycarbonyl or ethoxycarbonyl).

Preferably, each R¹⁶ independently represents halogen (particularly chlorine or fluorine), cyano, C₁-C₄ alkoxy (especially methoxy), C₁-C₄ alkoxycarbonyl (especially methoxycarbonyl), C₁-C₄ haloalkyl (especially trifluoromethyl), C₁-C₄ alkylcarbonyl (particularly methylcarbonyl), phenyl or C₁-C₄ alkyl (e.g. methyl or tert-butyl). Each R¹⁶ is especially a halogen atom or methyl group.

R¹⁷ represents a C₁-C₆, preferably C₁-C₄, alkyl group (e.g. methyl, ethyl, n-propyl, isopropyl, n-butyl, isobutyl, tert-butyl, n-pentyl or n-hexyl), amino or phenyl group.

R¹⁸ and R¹⁹ each independently represent a hydrogen atom or a C₁-C₆, preferably C₁-C₄, alkyl group (e.g. methyl, ethyl, n-propyl, isopropyl, n-butyl, isobutyl, tert-butyl, n-pentyl or n-hexyl), or R¹⁹ and R²⁰ together with the nitrogen atom to which they are attached form a 4- to 7-membered saturated heterocycle.

R²⁰ and R²¹ each independently represent a hydrogen atom or a C₁-C₆, preferably C₁-C₄, alkyl group (e.g. methyl, ethyl, n-propyl, isopropyl, n-butyl, isobutyl, tert-butyl, n-pentyl or n-hexyl) optionally substituted by a C₁-C₆, preferably C₁-C₄, alkoxycarbonyl substituent group.

R²² represents a hydrogen atom or a C₁-C₆, preferably C₁-C₄, alkyl group (e.g. methyl, ethyl, n-propyl, isopropyl, n-butyl, isobutyl, tert-butyl, n-pentyl or n-hexyl).

R²³ represents a hydrogen atom, or a C₁-C₆, preferably C₁-C₄, alkyl group (e.g. methyl, ethyl, n-propyl, isopropyl, n-butyl, isobutyl, tert-butyl, n-pentyl or n-hexyl) optionally substituted by carboxyl, C₁-C₆, preferably C₁-C₄, alkoxy or C₁-C₆, preferably C₁-C₄, alkoxycarbonyl.

Preferred compounds of the invention include:
*N*-(5-Chloro-2-{3-[3-(4-chloro-phenoxy)-pyrrolidin-1-yl]-2-hydroxy-propoxy}-phenyl)-isobutyramide,
Thiophene-2-carboxylic acid (2-{3-[3-(4-chloro-phenoxy)-pyrrolidin-1-yl]-2-hydroxy-propoxy}-phenyl)-amide,
N-[(2-{3-[3-(4-Chloro-phenoxy)-pyrrolidin-1-yl]-2-hydroxy-propoxy}-phenylcarbamoyl)-methyl]-benzamide,
Pyrazine-2-carboxylic acid (2-{3-[3-(4-chloro-phenoxy)-pyrrolidin-1-yl]-2-hydroxy-propoxy}-phenyl)-amide,
Cyclohexanecarboxylic acid (2-{3-[3-(4-chloro-phenoxy)-pyrrolidin-1-yl]-2-hydroxy-propoxy}-phenyl)-amide,
N-(2-{3-[3-(4-Chloro-phenoxy)-pyrrolidin-1-yl]-2-hydroxy-propoxy}-phenyl)-phthalamic acid methyl ester,
N-(2-{3-[3-(4-Chloro-phenoxy)-pyrrolidin-1-yl]-2-hydroxy-propoxy}-phenyl)-3-hydroxy-butyramide,
N-(2-{3-[3-(4-Chloro-phenoxy)-pyrrolidin-1-yl]-2-hydroxy-propoxy}-phenyl)-2-ureido-acetamide,
4-Acetylamino-N-(2-{3-[3-(4-chloro-phenoxy)-pyrrolidin-1-yl]-2-hydroxy-propoxy}-phenyl)-butyramide,
1-Acetyl-piperidine-4-carboxylic acid (2-{3-[3-(4-chloro-phenoxy)-pyrrolidin-1-yl]-2-hydroxy-propoxy}-phenyl)-amide,
N-(2-{3-[3-(4-Chloro-phenoxy)-pyrrolidin-1-yl]-2-hydroxy-propoxy}-phenyl)-3-methoxy-benzamide,
2-Acetylamino-N-(2-{3-[3-(4-chloro-phenoxy)-pyrrolidin-1-yl]-2-hydroxy-propoxy}-phenyl)-3-methyl-butyramide,
2-Acetylamino-N-(2-{3-[3-(4-chloro-phenoxy)-pyrrolidin-1-yl]-2-hydroxy-propoxy}-phenyl)-3-hydroxy-butyramide,
Adamantane-1-carboxylic acid (2-{3-[3-(4-chloro-phenoxy)-pyrrolidin-1-yl]-2-hydroxy-propoxy}-phenyl)-amide,
2-Acetylamino-N-(2-{3-[3-(4-chloro-phenoxy)-pyrrolidin-1-yl]-2-hydroxy-propoxy}-phenyl)-3-phenyl-propionamide,
N-(2-{3-[3-(4-Chloro-phenoxy)-pyrrolidin-1-yl]-2-hydroxy-propoxy}-phenyl)-2-methoxy-benzamide,
5-Methyl-thiophene-2-carboxylic acid (2-{3-[3-(4-chloro-phenoxy)-pyrrolidin-1-yl]-2-hydroxy-propoxy}-phenyl)-amide,
1-Acetyl-pyrrolidine-2-carboxylic acid (2-{3-[3-(4-chloro-phenoxy)-pyrrolidin-1-yl]-2-hydroxy-propoxy}-phenyl)-amide,
1,5-Dimethyl-1H-pyrazole-3-carboxylic acid (2-{3-[3-(4-chloro-phenoxy)-pyrrolidin-1-yl]-2-hydroxy-propoxy}-phenyl)-amide,
5-Oxo-pyrrolidine-2-carboxylic acid (2-{3-[3-(4-chloro-phenoxy)-pyrrolidin-1-yl]-2-hydroxy-propoxy}-phenyl)-amide,
1H-Indole-6-carboxylic acid (2-{3-[3-(4-chloro-phenoxy)-pyrrolidin-1-yl]-2-hydroxy-propoxy}-phenyl)-amide,
Cyclobutanecarboxylic acid (2-{3-[3-(4-chloro-phenoxy)-pyrrolidin-1-yl]-2-hydroxy-propoxy}-phenyl)-amide,
N-(2-{3-[3-(4-Chloro-phenoxy)-pyrrolidin-1-yl]-2-hydroxy-propoxy}-phenyl)-propionamide,
Pentanoic acid (2-{3-[3-(4-chloro-phenoxy)-pyrrolidin-1-yl]-2-hydroxy-propoxy}-phenyl)-amide,
Pent-4-enoic acid (2-{3-[3-(4-chloro-phenoxy)-pyrrolidin-1-yl]-2-hydroxy-propoxy}-phenyl)-amide,
Cyclopentanecarboxylic acid (2-{3-[3-(4-chloro-phenoxy)-pyrrolidin-1-yl]-2-hydroxy-propoxy}-phenyl)-amide,
Cyclopropanecarboxylic acid (2-{3-[3-(4-chloro-phenoxy)-pyrrolidin-1-yl]-2-hydroxy-propoxy}-phenyl)-amide,
N-(2-{3-[3-(4-Chloro-phenoxy)-pyrrolidin-1-yl]-2-hydroxy-propoxy}-phenyl)-isobutyramide,
N-(2-{3-[3-(4-Chloro-phenoxy)-pyrrolidin-1-yl]-2-hydroxy-propoxy}-phenyl)-2-methylsulfanyl-acetamide,
2-Acetylamino-N-(2-{3-[3-(4-chloro-phenoxy)-pyrrolidin-1-yl]-2-hydroxy-propoxy}-phenyl)-propionamide,
N-(2-{3-[3-(4-Chloro-phenoxy)-pyrrolidin-1-yl]-2-hydroxy-propoxy}-phenyl)-butyramide,
N-(2-{3-[3-(4-Chloro-phenoxy)-pyrrolidin-1-yl]-2-hydroxy-propoxy}-phenyl)-3-methyl-butyramide,
N-(2-{3-[3-(4-Chloro-phenoxy)-pyrrolidin-1-yl]-2-hydroxy-propoxy}-phenyl)-2-methoxy-acetamide,
N-(2-{3-[3-(4-Chloro-phenoxy)-pyrrolidin-1-yl]-2-hydroxy-propoxy}-phenyl)-2,2-dimethyl-propionamide,
5-Oxo-hexanoic acid (2-{3-[3-(4-chloro-phenoxy)-pyrrolidin-1-yl]-2-hydroxy-propoxy}-phenyl)-amide,
Hexanoic acid (2-{3-[3-(4-chloro-phenoxy)-pyrrolidin-1-yl]-2-hydroxy-propoxy}-phenyl)-amide,
2-Chloro-N-(2-{3-[3-(4-chloro-phenoxy)-pyrrolidin-1-yl]-2-hydroxy-propoxy}-phenyl)-benzamide,
3-Chloro-N-(2-{3-[3-(4-chloro-phenoxy)-pyrrolidin-1-yl]-2-hydroxy-propoxy}-phenyl)-benzamide,
(4*R*)-*N*-(2-{3-[3-(4-chlorophenoxy)-1-pyrrolidinyl]-2-hydroxypropoxy}phenyl)-1,3-thiazolidine-4-carboxamide ditrifluoroacetate,
Thiophene-2-carboxylic acid (2-{3-[4-(3,4-dichloro-phenoxy)-piperidin-1-yl]-2-hydroxy-propoxy}-phenyl)-amide,
N-(2-{3-[4-(3,4-Dichloro-phenoxy)-piperidin-1-yl]-2-hydroxy-propoxy}-phenyl)-benzamide,
N-(2-{3-[4-(3,4-Dichloro-phenoxy)-piperidin-1-yl]-2-hydroxy-propoxy}-phenyl)-nicotinamide,
Pyridine-2-carboxylic acid (2-{3-[4-(3,4-dichloro-phenoxy)-piperidin-1-yl]-2-hydroxy-propoxy}-phenyl)-amide,
N-(2-{3-[4-(3,4-Dichloro-phenoxy)-piperidin-1-yl]-2-hydroxy-propoxy}-phenyl)-isonicotinamide,
Cyclohexanecarboxylic acid (2-{3-[4-(3,4-dichloro-phenoxy)-piperidin-1-yl]-2-hydroxy-propoxy} -phenyl)-amide,
N-(2- {3-[4-(3,4-Dichloro-phenoxy)-piperidin-1-yl]-2-hydroxy-propoxy}-phenyl)-3-hydroxy-butyramide,
5-Methyl-thiophene-2-carboxylic acid (2-{3-[4-(3,4-dichloro-phenoxy)-piperidin-1-yl]-2-hydroxy-propoxy} -phenyl)-amide,
Cyclobutanecarboxylic acid (2-{3-[4-(3,4-dichloro-phenoxy)-piperidin-1-yl]-2-hydroxy-propoxy}-phenyl)-amide,
N-(2-{3-[4-(3,4-Dichloro-phenoxy)-piperidin-1-yl]-2-hydroxy-propoxy}-phenyl)-propionamide,
Pentanoic acid (2-{3-[4-(3,4-dichloro-phenoxy)-piperidin-1-yl]-2-hydroxy-propoxy}-phenyl)-amide,
Pent-4-enoic acid (2-{3-[4-(3,4-dichloro-phenoxy)-piperidin-1-yl]-2-hydroxy-propoxy}-phenyl)-amide,
Cyclopentanecarboxylic acid (2-{3-[4-(3,4-dichloro-phenoxy)-piperidin-1-yl]-2-hydroxy-propoxy} -phenyl)-amide,
N-(2-{3-[4-(3,4-Dichloro-phenoxy)-piperidin-1-yl]-2-hydroxy-propoxy}-phenyl)-3-methyl-butyramide,
*N*-(2-{3-[3-(4-chlorophenoxy)-1-pyrrolidinyl]-2-hydroxypropoxy}phenyl)-2,2,2-trifluoroacetamide hydrochloride,
4-(2-{3-[4-(3,4-Dichloro-phenoxy)-piperidin-1-yl]-2-hydroxy-propoxyl-phenylcarbamoyl)-3-methyl-butyric acid,
N-(2-{3-[4-(3,4-Dichloro-phenoxy)-piperidin-1-yl]-2-hydroxy-propoxy}-phenyl)-succinamic acid,
Furan-2-carboxylic acid (2-{3-[3-(4-chloro-phenoxy)-pyrrolidin-1-yl]-2-hydroxy-propoxy}-4-methyl-phenyl)-amide,
1H-Pyrrole-2-carboxylic acid (2-{3-[3-(4-chloro-phenoxy)-pyrrolidin-1-yl]-2-hydroxy-propoxy}-4-methyl-phenyl)-amide,
Thiophene-2-carboxylic acid (2-{3-[3-(4-chloro-phenoxy)-pyrrolidin-1-yl]-2-hydroxy-propoxy}-4-methyl-phenyl)-amide,
Cyclopentanecarboxylic acid (2-{3-[3-(4-chloro-phenoxy)-pyrrolidin-1-yl]-2-hydroxy-propoxy}-4-methyl-phenyl)-amide,
5-Methyl-thiophene-2-carboxylic acid (2-{3-[3-(4-chloro-phenoxy)-pyrrolidin-1-yl]-2-hydroxy-propoxy}-4-methyl-phenyl)-amide,
3-Chloro-thiophene-2-carboxylic acid (2-{3-[3-(4-chloro-phenoxy)-pyrrolidin-1-yl]-2-hydroxy-propoxy}-4-methyl-phenyl)-amide,
5-Methyl-isoxazole-4-carboxylic acid (2-{3-[3-(4-chloro-phenoxy)-pyrrolidin-1-yl]-2-hydroxy-propoxy}-4-methyl-phenyl)-amide,
[1,2,3]Thiadiazole-4-carboxylic acid (2-{3-[3-(4-chloro-phenoxy)-pyrrolidin-1-yl]-2-hydroxy-propoxy}-4-methyl-phenyl)-amide,
3-Methyl-furan-2-carboxylic acid (2-{3-[3-(4-chloro-phenoxy)-pyrrolidin-1-yl]-2-hydroxy-propoxy}-4-methyl-phenyl)-amide,
Cyclopent-1-enecarboxylic acid (2-{3-[3-(4-chloro-phenoxy)-pyrrolidin-1-yl]-2-hydroxy-propoxy}-4-methyl-phenyl)-amide,
2-Methyl-furan-3-carboxylic acid (2-{3-[3-(4-chloro-phenoxy)-pyrrolidin-1-yl]-2-hydroxy-propoxy}-4-methyl-phenyl)-amide,
3-Methyl-thiophene-2-carboxylic acid (2-{3-[3-(4-chloro-phenoxy)-pyrrolidin-1-yl]-2-hydroxy-propoxy}-4-methyl-phenyl)-amide,
5-Nitro-1H-pyrazole-3-carboxylic acid (2-{3-[3-(4-chloro-phenoxy)-pyrrolidin-1-yl]-2-hydroxy-propoxy}-4-methyl-phenyl)-amide,
Thiophene-3-carboxylic acid (2-{3-[3-(4-chloro-phenoxy)-pyrrolidin-1-yl]-2-hydroxy-propoxy}-4-methyl-phenyl)-amide,
Cyclobutanecarboxylic acid (2-{3-[3-(4-chloro-phenoxy)-pyrrolidin-1-yl]-2-hydroxy-propoxy}-4-methyl-phenyl)-amide,
Furan-2-carboxylic acid (2-{3-[3-(4-fluoro-phenoxy)-pyrrolidin-1-yl]-2-hydroxy-propoxy}-4-methyl-phenyl)-amide,
1H-Pyrrole-2-carboxylic acid (2-{3-[3-(4-fluoro-phenoxy)-pyrrolidin-1-yl]-2-hydroxy-propoxy}-4-methyl-phenyl)-amide,
Thiophene-2-carboxylic acid (2-{3-[3-(4-fluoro-phenoxy)-pyrrolidin-1-yl]-2-hydroxy-propoxy}-4-methyl-phenyl)-amide,
3-Chloro-thiophene-2-carboxylic acid (2-{3-[3-(4-fluoro-phenoxy)-pyrrolidin-1-yl]-2-hydroxy-propoxy} -4-methyl-phenyl)-amide,
5-Methyl-isoxazole-4-carboxylic acid (2-{3-[3-(4-fluoro-phenoxy)-pyrrolidin-1-yl]-2-hydroxy-propoxy}-4-methyl-phenyl)-amide,
3-Methyl-furan-2-carboxylic acid (2-{3-[3-(4-fluoro-phenoxy)-pyrrolidin-1-yl]-2-hydroxy-propoxy}-4-methyl-phenyl)-amide,
Cyclopent-1-enecarboxylic acid (2-{3-[3-(4-fluoro-phenoxy)-pyrrolidin-1-yl]-2-hydroxy-propoxy}-4-methyl-phenyl)-amide,
2-Methyl-furan-3-carboxylic acid (2-{3-[3-(4-fluoro-phenoxy)-pyrrolidin-1-yl]-2-hydroxy-propoxy}-4-methyl-phenyl)-amide,
3-Methyl-thiophene-2-carboxylic acid (2-{3-[3-(4-fluoro-phenoxy)-pyrrolidin-1-yl]-2-hydroxy-propoxy}-4-methyl-phenyl)-amide,
5-Chloro-thiophene-2-carboxylic acid (2-{3-[3-(4-fluoro-phenoxy)-pyrrolidin-1-yl]-2-hydroxy-propoxy}-4-methyl-phenyl)-amide,
Thiophene-3-carboxylic acid (2-{3-[3-(4-fluoro-phenoxy)-pyrrolidin-1-yl]-2-hydroxy-propoxy}-4-methyl-phenyl)-amide,
2,5-Dimethyl-furan-3-carboxylic acid (2-{3-[3-{4-fluoro-phenoxy)-pyrrolidin-1-yl]-2-hydroxy-propoxy}-4-methyl-phenyl)-amide,
Cyclobutanecarboxylic acid (2-{3-[3-(4-fluoro-phenoxy)-pyrrolidin-1-yl]-2-hydroxy-propoxy}-4-methyl-phenyl)-amide,
Furan-3-carboxylic acid (2-{3-[3-(4-fluoro-phenoxy)-pyrrolidin-1-yl]-2-hydroxy-propoxy}-4-methyl-phenyl)-amide,
N-{2-[(3-{3-[(4-fluorophenyl)oxy]-1-pyrrolidinyl}-2-hydroxypropyl)oxy]-4-methylphenyl}-1H-pyrrole-2-carboxamide,
N-{2-[(3-{3-[(4-chlorophenyl)oxy]-1-pyrrolidinyl}-2-hydroxypropyl)oxy]-4-methylphenyl}-3-thiophenecarboxamide,
*N*-{2-[(3-{3-[(4-chlorophenyl)oxy]-1-pyrrolidinyl}-2-hydroxy-2-methylpropyl)oxy]phenyl}-2-thiophenecarboxamide, compound with trifluoroacetic acid,
N-{2-[(3-{3-[(4-fluorophenyl)oxy]-1-pyrrolidinyl}-2-hydroxypropyl)oxy]-4-methylphenyl}-2-thiophenecarboxamide,
N-{2-[(3-{3-[(4-chlorophenyl)oxy]-1-pyrrolidinyl}-2-hydroxypropyl)oxy]phenyl}-2-furancarboxamide,
N-{2-[(3-{3-[(4-chlorophenyl)oxy]-1-pyrrolidinyl}-2-hydroxypropyl)oxy]phenyl}-1-pyrrole-2-carboxamide,
N-{2-[(3-{3-[(4-chlorophenyl)oxy]-1-pyrrolidinyl}-2-hydroxypropyl)oxy]-4-methylphenyl}-1H-pyrrole-3-carboxamide,
N-{2-[(3-{3-[(4-fluorophenyl)oxy]-1-pyrrolidinyl}-2-hydroxypropyl)oxy]-4-methylphenyl}-2-furancarboxamide,
N-{2-[(3-{3-[(4-chlorophenyl)oxy]-1-pyrrolidinyl}-2-hydroxy-2-methylpropyl)oxy]phenyl}cyclopentanecarboxamide, compound with trifluoracetic acid,
N-(2-{3-[3-(4-Fluoro-phenoxy)-pyrrolidin-1-yl]-2-hydroxy-2-methyl-propoxy}-phenyl)-benzamide,
N-(2-{3-[3-(4-Cyano-phenoxy)-pyrrolidin-1-yl]-2-hydroxy-2-methyl-propoxy}-phenyl)-benzamide,
N-(2-{3-[4-(3,4-Dichloro-phenoxy)-piperidin-1-yl]-2-hydroxy-2-methyl-propoxy}-phenyl)-benzamide,
N-(2-{3-[3-(4-Chloro-phenoxy)-pyrrolidin-1-yl]-2-hydroxy-2-methyl-propoxy}-phenyl)-benzamide, and
N-(2-{3-[4-(3,4-Dichloro-phenylamino)-piperidin-1-yl]-2-hydroxy-2-methyl-propoxy}-phenyl)-benzamide.

The present invention further provides a process for the preparation of a compound of formula (I) as defined above which comprises reacting a compound of general formula 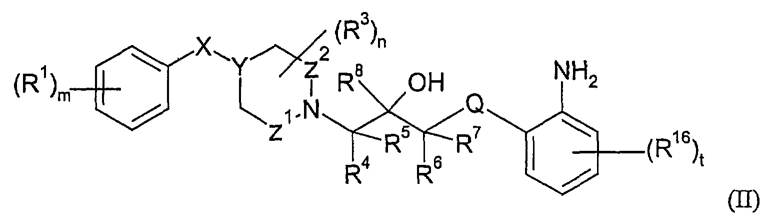 or a salt thereof (e.g. an acid addition salt such as a hydrochloride salt), wherein m, n, t, R¹, R³, R⁴, R⁵, R⁶, R⁷, R⁸, R¹⁶, Q, Z¹ and Z² are as defined in formula (I), with a compound of general formula

R¹⁵ - CO₂H (III)

or chemically equivalent derivative thereof (e.g. acyl halide or anhydride derivative) wherein R¹⁵ is as defined in formula (I);
and optionally thereafter forming a pharmaceutically acceptable salt or solvate of the compound of formula (I) obtained.

The process of the invention may conveniently be carried out in a solvent, e.g. an organic solvent such as an alcohol (e.g. methanol or ethanol), a hydrocarbon (e.g. toluene), an amine (e.g. triethylamine or diisopropylethylamine) or acetonitrile at a temperature of, for example, 15°C or above, such as a temperature in the range from 20 to 120°C.

Compounds of formulae (II) and (III) are either commercially available, are well known in the literature or may be prepared easily using known techniques.

It will be appreciated by those skilled in the art that in the process of the present invention certain functional groups such as hydroxyl or amino groups in the starting reagents or intermediate compounds may need to be protected by protecting groups. Thus, the preparation of the compounds of formula (I) may involve, at an appropriate stage, the removal of one or more protecting groups.

The protection and deprotection of functional groups is described in 'Protective Groups in Organic Chemistry', edited by J.W.F. McOmie, Plenum Press (1973) and 'Protective Groups in Organic Synthesis', 2nd edition, T.W. Greene and P.G.M. Wuts, Wiley-Interscience (1991).

The compounds of formula (I) above may be converted to a pharmaceutically acceptable salt or solvate thereof, preferably an acid addition salt such as a hydrochloride, hydrobromide, phosphate, acetate, fumarate, maleate, tartrate, citrate, oxalate, methanesulphonate or *p*-toluenesulphonate.

Compounds of formula (I) are capable of existing in stereoisomeric forms. It will be understood that the invention encompasses the use of all geometric and optical isomers of the compounds of formula (I) and mixtures thereof including racemates. The use of tautomers and mixtures thereof also form an aspect of the present invention.

The compounds of formula (I) have activity as pharmaceuticals, in particular as modulators of chemokine receptor (especially MIP-1α chemokine receptor) activity, and may be used in the treatment of autoimmune, inflammatory, proliferative and hyperproliferative diseases and immunologically-mediated diseases including rejection of transplanted organs or tissues and Acquired Immunodeficiency Syndrome (AIDS).

Examples of these conditions are:
(1) **(the respiratory tract**) airways diseases including chronic obstructive pulmonary disease (COPD) such as irreversible COPD; asthma, such as bronchial, allergic, intrinsic, extrinsic and dust asthma, particularly chronic or inveterate asthma (e.g. late asthma and airways hyper-responsiveness); bronchitis; acute, allergic, atrophic rhinitis and chronic rhinitis including rhinitis caseosa, hypertrophic rhinitis, rhinitis purulenta, rhinitis sicca and rhinitis medicamentosa; membranous rhinitis including croupous, fibrinous and pseudomembranous rhinitis and scrofoulous rhinitis; seasonal rhinitis including rhinitis nervosa (hay fever) and vasomotor rhinitis; sarcoidosis, farmer's lung and related diseases, fibroid lung and idiopathic interstitial pneumonia;
(2) **(bone and joints)** rheumatoid arthritis, seronegative spondyloarthropathies (including ankylosing spondylitis, psoriatic arthritis and Reiter's disease), Behcet's disease, Sjogren's syndrome and systemic sclerosis;
(3) **(skin)** psoriasis, atopical dermatitis, contact dermatitis and other eczmatous dermitides, seborrhoetic dermatitis, Lichen planus, Pemphigus, bullous Pemphigus, Epidermolysis bullosa, urticaria, angiodermas, vasculitides, erythemas, cutaneous eosinophilias, uveitis, Alopecia areata and vernal conjunctivitis;
(4) **(gastrointestinal tract)** Coeliac disease, proctitis, eosinopilic gastro-enteritis, mastocytosis, Crohn's disease, ulcerative colitis, food-related allergies which have effects remote from the gut, e.g., migraine, rhinitis and eczema;
(5) **(other tissues and systemic disease)** multiple sclerosis, atherosclerosis, Acquired Immunodeficiency Syndrome (AIDS), lupus erythematosus, systemic lupus, erythematosus, Hashimoto's thyroiditis, myasthenia gravis, type I diabetes, nephrotic syndrome, eosinophilia fascitis, hyper IgE syndrome, lepromatous leprosy, sezary syndrome and idiopathic thrombocytopenia pupura;
(6) **(allograft rejection)** acute and chronic following, for example, transplantation of kidney, heart, liver, lung, bone marrow, skin and cornea; and chronic graft versus host disease;
(7) cancers, especially non-small cell lung cancer (NSCLC) and squamous sarcoma;
(8) diseases in which angiogenesis is associated with raised chemokine levels (e.g. NSCLC); and
(9) cystic fibrosis, stroke, re-perfusion injury in the heart, brain, peripheral limbs and sepsis.

Thus, the present invention provides a compound of formula (I), or a pharmaceutically-acceptable salt or solvate thereof, as hereinbefore defined for use in therapy.

In a further aspect, the present invention provides the use of a compound of formula (I), or a pharmaceutically acceptable salt or solvate thereof, as hereinbefore defined in the manufacture of a medicament for use in therapy.

In the context of the present specification, the term "therapy" also includes "prophylaxis" unless there are specific indications to the contrary. The terms "therapeutic" and "therapeutically" should be construed accordingly.

The invention also provides a method of treating an inflammatory disease in a patient suffering from, or at risk of, said disease, which comprises administering to the patient a therapeutically effective amount of a compound of formula (I), or a pharmaceutically acceptable salt or solvate thereof, as hereinbefore defined.

The invention still further provides a method of treating an airways disease in a patient suffering from, or at risk of, said disease, which comprises administering to the patient a therapeutically effective amount of a compound of formula (I), or a pharmaceutically acceptable salt or solvate thereof, as hereinbefore defined.

For the above-mentioned therapeutic uses the dosage administered will, of course, vary with the compound employed, the mode of administration, the treatment desired and the disorder indicated. The daily dosage of the compound of formula (I) may be in the range from 0.001 mg/kg to 30 mg/kg.

The compounds of formula (I) and pharmaceutically acceptable salts and solvates thereof may be used on their own but will generally be administered in the form of a pharmaceutical composition in which the formula (I) compound/salt/solvate (active ingredient) is in association with a pharmaceutically acceptable adjuvant, diluent or carrier. Depending on the mode of administration, the pharmaceutical composition will preferably comprise from 0.05 to 99 %w (per cent by weight), more preferably from 0.05 to 80 %w, still more preferably from 0.10 to 70 %w, and even more preferably from 0.10 to 50 %w, of active ingredient, all percentages by weight being based on total composition.

The present invention also provides a pharmaceutical composition comprising a compound of formula (I), or a pharmaceutically acceptable salt or solvate thereof, as hereinbefore defined, in association with a pharmaceutically acceptable adjuvant, diluent or carrier.

The invention further provides a process for the preparation of a pharmaceutical composition of the invention which comprises mixing a compound of formula (I), or a pharmaceutically acceptable salt or solvate thereof, as hereinbefore defined, with a pharmaceutically acceptable adjuvant, diluent or carrier.

The pharmaceutical compositions may be administered topically (e.g. to the lung and/or airways or to the skin) in the form of solutions, suspensions, heptafluoroalkane aerosols and dry powder formulations; or systemically, e.g. by oral administration in the form of tablets, capsules, syrups, powders or granules, or by parenteral administration in the form of solutions or suspensions, or by subcutaneous administration or by rectal administration in the form of suppositories or transdermally.

The invention will now be further explained by reference to the following illustrative examples, in which ¹H NMR spectra were recorded on Varian Unity Inova 400. The central solvent peak of chloroform-*d* (δ_{H} 7.27 ppm) were used as internal standard. Low resolution mass spectra and accurate mass determination were recorded on a Hewlett-Packard 1100 LC-MS system equipped with APCI /ESI ionisation chambers.
All solvents and commercial reagents were laboratory grade and used as received.
The nomenclature used for the compounds was generated with ACD/IUPAC Name Pro. The following abbreviations are used in the examples:

| | |
|---|---|
| NMP | 1-Methyl-2-pyrrolidinone |
| DIEA | N,N-Diisopropylethylamine |
| HBTU | 2-(1H-Benzotriazol-1-yl}-1,1,3,3-tetramethyluronium hexafluorophosphate |
| HoBT | 1-Hydroxybenzotriazole |
| THF | Tetrahydrofuran |

### Example 1

### N-(5-Chloro-2-{3-[3-(4-chloro-phenoxy)-pyrrolidin-1-yl]-2-hydroxy-propoxy}-phenyl)-isobutyramide

**a) *N*-(5-Chloro-2-hydroxy-phenyl)-isobutyramide**
   In a flask was added 4-chloro-2-aminophenol (1.2 g, 8.39 mmole) and water (25 ml).
   The suspension was vigorously stirred and isobutyric anhydride (1.6 ml, 10.5 mmole) was added. The mixture was heated to 60°C for 30 minutes under vigorous stirring. The emulsion was cooled, and a precipitate was formed, which was collected through filtration. The solid was washed twice with water on the filter and was finally dried to give 1.4 g (78%) of the sub-title compound as a white solid.
   ¹H-NMR (400 MHz, DMSO-*d*_{*6*}) δ: 10.11 (1H, s); 9.12 (1H, s); 7.94 (1H, d, *J* 2.5 Hz); 6.95 (1H, dd, *J* 8.7 2.6 Hz); 6.84 (1H, d, *J* 8.5 Hz); 2.79 (1H, p, *J* 6.7 Hz); 1.08 (6H, d, *J* 6.8 Hz)
**b) *N*-(5-Chloro-2-oxiranylmethoxy-phenyl)-isobutyramide**
   In a vial was added the compound obtained in a) (0.4 g, 1.87 mmole), epibromohydrin (0.28 g, 2.06 mmole), K₂CO₃ (0.5 g, 3.7 mmole) and DMF (2 ml). The vial was sealed and heated with stirring (2 hours, 60°C). The mixture was then partitioned between EtOAc and water, and the organic phase was washed twice with water and once with brine, and was finally evaporated to give a brown solid. The crude epoxide was purified on silica, to give 0.22 g (44%) of the sub-title compound as a white solid.
**c)** In a vial was added the compound obtained in b) (0.026 g, 0.13 mmole), 3-(4-chlorophenoxy)-pyrrolidine (0.035 g, 0.13 mmole) in ethanol (2 ml). The vial was sealed and heated with stirring at 75°C for 3 hours. The solution was allowed to cool, and the solvent was evaporated. The crude product was purified on silica, and the pure fractions were collected. The title compound was lyophilized as the hydrochloride, giving 0.055 g (84%) as a white solid. The compound was a mixture of four stereoisomers, which had an effect on the NMR-spectra.
   ¹H-NMR (400 MHz, DMSO-*d*_{*6*}) δ: 10.84-10.34 (1H, m); 9.12 (1H, s); 8.09 (1H, s); 7.36 (2H, dd, *J* 9.2 1.3 Hz); 7.11-7.00 (3H, m); 7.00 (2H, d, *J* 8.8 Hz); 6.22-6.06 (1H, m); 5.22-5.10 (1H, m); 4.34 (1H, bs); 4.08-3.96 (1.5H, m); 3.95-3.87 (1H, m); 3.83-3.66 (1.5H, m); 3.61-3.23 (3H, m); 2.86 (1H, sept, *J* 6.6 Hz); 2.64-2.51 (½H, m); 2.36-2.14 (1H, m); 2.14-2.00 (½H, m); 1.08 (6H, d, *J* 6.7 Hz)
   APCI-MS: m/z 467.2 [MH+]

### Aniline Intermediate 1

### 1-(2-aminophenoxy)-3-[4-(3,4-dichlorophenoxy)-1-piperidinyl]-2-propanol dihydrochloride

N-(2-{3-[4-(3,4-dichlorophenoxy)-1-piperidinyl]-2-hydroxypropoxy}phenyl)acetamide (1.418g, 3.13 mmol, prepared by analogy to Example 1) was dissolved in 50ml HCl (35%/aq, puriss) and refluxed overnight. The product precipitated and was filtered and dried to give 0.835 g (65%) of the title compound.

APCI-MS m/z: 411, 413 [MH⁺]
¹H NMR (400 MHz, CDCl₃) : δ 8.39-3.31 (m, 2H), 7.31(d, 1H), 7.01-6.98(m, 3H), 6.94-6.91(m, 1H), 6.75(dd, 1H), 4.31(m, 1H), 4.12-4.02 (m, 2H), 3.92(dd, 1H), 2.90(m, 1H), 2.69(m, 1H), 2.62-2.51(m, 2H), 2.46(dd, 1H), 2.34(m, 1H), 2.18(s, 3H), 2.04-1.93(m, 2H), 1.89-1.77(m, 2H).

### Aniline Intermediate 2

### 1-[(2-aminophenyl)oxy]-3-{3-[(4-chlorophenyl)oxy]-1-pyrrolidinyl}-2-propanol dihydrochloride

Prepared according to the method described in Aniline Intermediate 1.
APCI-MS m/z: 363,365 [MH⁺]

The intermediate anilines 1 and 2 described above were used in the following examples.

### Example 2

### Thiophene-2-carboxylic acid (2-{3-[3-(4-chloro-phenoxy)-pyrrolidin-1-yl]-2-hydroxy-propoxy}-phenyl)-amide

To a solution of 80 uL 0.2M 2-thiophenecarboxylic acid in NMP were HBTU (80uL, 0.2M/NMP) ,HoBT (80uL, 0.2M/NMP), DIEA (30uL, 0.5M/NMP) and pyridine (30uL, 0.5M/NMP) added and stirred for 30 minutes before 1-[(2-aminophenyl)oxy]-3-{3-[(4-chlorophenyl)oxy]-1-pyrrolidinyl}-2-propanol (75uL, 0.2M/NMP) was added. The mixture was stirred overnight at roomtemperature before it was concentrated under reduced pressure to dryness. The product was diluted with 1000uL dichloromethane and washed with with sat.NaHCO₃/aq (800uL), 1.8%HCl/aq(800uL) and sat. NaCl/aq.
The organic layer was concentrated under reduced pressure to dryness and used without further purification. Yield 3.6mg, 51%

APCI-MS m/z: 473.2 [MH⁺]
¹H NMR (400 MHz, CD₃OD) : δ 7.88-7.85 (d, 1H), 7.74-7.65 (m, 2H), 7.34-7.28 (m, 2H), 7.27-7.21(m, 1H), 7.20-7.15 (m, 1H), 7.14-7.09 (dd, 1H), 7.06-7.00 (m, 1H), 6.96-6.91 (m, 2H), 5.18-5.12 (m, 1H), 4.39-4.30 (m, 1H), 4.19-3.24 (m, 9H), 2.66-2.11 (m, 3H)

The following Examples 3 to 53 were prepared by methods analogous to the method described in Example 2.

### Example 3

### N-[(2-{3-[3-(4-Chloro-phenoxy)-pyrrolidin-1-yl]-2-hydroxy-propoxy}-phenylcarbamoyl)-methyl]-benzamide

APCI-MS m/z: 524.3 [MH⁺]

### Example 4

### Pyrazine-2-carboxylic acid (2-{3-[3-(4-chloro-phenoxy)-pyrrolidin-1-yl]-2-hydroxy-propoxy}-phenyl)-amide

APCI-MS m/z: 469.2 [MH⁺]

### Example 5

### Cyclohexanecarboxylic acid (2-{3-[3-(4-chloro-phenoxy)-pyrrolidin-1-yl]-2-hydroxy-propoxy}-phenyl)-amide

APCI-MS m/z: 473.3 [MH⁺]

### Example 6

### N-(2-{3-[3-(4-Chloro-phenoxy)-pyrrolidin-1-yl]-2-hydroxy-propoxy}-phenyl)-phthalamic acid methyl ester

APCI-MS m/z: 525.2 [MH⁺]

### Example 7

### N-(2-{3-[3-(4-Chloro-phenoxy)-pyrrolidin-1-yl]-2-hydroxy-propoxy}-phenyl)-3-hydroxy-butyramide

APCI-MS m/z: 449.2 [MH⁺]

### Example 8

### N-(2-{3-[3-(4-Chloro-phenoxy)-pyrrolidin-1-yl]-2-hydroxy-propoxy}-phenyl)-2-ureido-acetamide

APCI-MS m/z: 463.2 [MH⁺]

### Example 9

### 4-Acetylamino-N-(2-{3-[3-(4-chloro-phenoxy)-pyrrolidin-1-yl]-2-hydroxy-propoxy}-phenyl)-butyramide

APCI-MS m/z: 490.3 [MH⁺]

### Example 10

### 1-Acetyl-piperidine-4-carboxylic acid (2-{3-[3-(4-chloro-phenoxy)-pyrrolidin-1-yl]-2-hydroxy-propoxy}-phenyl)-amide

APCI-MS m/z: 516.3 [MH⁺]

### Example 11

### N-(2-{3-[3-(4-Chloro-phenoxy)-pyrrolidin-1-yl]-2-hydroxy-propoxy}-phenyl)-3-methoxy-benzamide

APCI-MS m/z: 497.2 [MH⁺]

### Example 12

### 2-Acetylamino-N-(2-{3-[3-(4-chloro-phenoxy)-pyrrolidin-1-yl]-2-hydroxy-propoxy}-phenyl)-3-methyl-butyramide

APCI-MS m/z: 504.3 [MH⁺]

### Example 13

### 2-Acetylamino-N-(2-{3-[3-(4-chloro-phenoxy)-pyrrolidin-1-yl]-2-hydroxy-propoxy}-phenyl)-3-hydroxy-butyramide

APCI-MS m/z: 506.2 [MH⁺]

### Example 14

### Adamantane-1-carboxylic acid (2-{3-[3-(4-chloro-phenoxy)-pyrrolidin-1-yl]-2-hydroxy-propoxy}-phenyl)-amide

APCI-MS m/z: 525.3 [MH⁺]

### Example 15

### 2-Acetylamino-N-(2-{3-[3-(4-chloro-phenoxy)-pyrrolidin-1-yl]-2-hydroxy-propoxy}-phenyl)-3-phenyl-propionamide

APCI-MS m/z: 552.3 [MH⁺]

### Example 16

### N-(2-{3-[3-(4-Chloro-phenoxy)-pyrrolidin-1-yl]-2-hydroxy-propoxy}-phenyl)-2-methoxy-benzamide

APCI-MS m/z: 497.2 [MH⁺]

### Example 17

### 5-Methyl-thiophene-2-carboxylic acid (2-{3-(3-(4-chloro-phenoxy)-pyrrolidin-1-yl]-2-hydroxy-propoxy}-phenyl)-amide

APCI-MS m/z: 487.2 [MH⁺]

### Example 18

### 1-Acetyl-pyrrolidine-2-carboxylic acid (2-{3-(3-(4-chloro-phenoxy)-pyrrolidin-1-yl]-2-hydroxy-propoxy}-phenyl)-amide

APCI-MS m/z: 502.3 [MH⁺]

### Example 19

### 1,5-Dimethyl-1H-pyrazole-3-carboxylic acid (2-{3-[3-(4-chloro-phenoxy)-pyrrolidin-1-yl]-2-hydroxy-propoxy}-phenyl)-amide

APCI-MS m/z: 485.3 [MH⁺]

### Example 20

### 5-Oxo-pyrrolidine-2-carboxylic acid (2-{3-[3-(4-chloro-phenoxy)-pyrrolidin-1-yl]-2-hydroxy-propoxy}-phenyl)-amide

APCI-MS m/z: 474.2 [MH⁺]

### Example 21

### 1H-Indole-6-carboxylic acid (2-{3-[3-(4-chloro-phenoxy)-pyrrolidin-1-yl]-2-hydroxy-propoxy}-phenyl)-amide

APCI-MS m/z: 506.2 [MH⁺]

### Example 22

### Cyclobutanecarboxylic acid (2-{3-[3-(4-chloro-phenoxy)-pyrrolidin-1-yl]-2-hydroxy-propoxy}-phenyl)-amide

APCI-MS m/z: 445.3 [MH⁺]

### Example 23

### N-(2-{3-[3-(4-Chloro-phenoxy)-pyrrolidin-1-yl]-2-hydroxy-propoxy}-phenyl)-propionamide

APCI-MS m/z: 419.2 [MH⁺]

### Example 24

### Pentanoic acid (2-{3-[3-(4-chloro-phenoxy)-pyrrolidin-1-yl]-2-hydroxy-propoxy}-phenyl)-amide

APCI-MS m/z: 447.3 [MH⁺]

### Example 25

### Pent-4-enoic acid (2-{3-[3-(4-chloro-phenoxy)-pyrrolidin-1-yl]-2-hydroxy-propoxy}-phenyl)-amide

APCI-MS m/z: 445.3 [MH⁺]

### Example 26

### Cyclopentanecarboxylic acid (2-{3-[3-(4-chloro-phenoxy)-pyrrolidin-1-yl]-2-hydroxy-propoxy}-phenyl)-amide

APCI-MS m/z: 459.3 [MH⁺]

### Example 27

### Cyclopropanecarboxylic acid (2-{3-[3-(4-chloro-phenoxy)-pyrrolidin-1-yl]-2-hydroxy-propoxy}-phenyl)-amide

APCI-MS m/z: 431.2 [MH⁺]

### Example 28

### N-(2-{3-[3-(4-Chloro-phenoxy)-pyrrolidin-1-yl]-2-hydroxy-propoxy}-phenyl)-isobutyramide

APCI-MS m/z: 433.3 [MH⁺]

### Example 29

### N-(2-{3-[3-(4-Chloro-phenoxy)-pyrrolidin-1-yl]-2-hydroxy-propoxy}-phenyl)-2-methylsulfanyl-acetamide

APCI-MS m/z: 451.2 [MH⁺]

### Example 30

### 2-Acetylamino-N-(2-{3-[3-(4-chloro-phenoxy)-pyrrolidin-1-yl]-2-hydroxy-propoxy}-phenyl)-propionamide

APCI-MS m/z: 476.2 [MH⁺]

### Example 31

### N-(2-{3-[3-(4-Chloro-phenoxy)-pyrrolidin-1-yl]-2-hydroxy-propoxy}-phenyl)-butyramide

APCI-MS m/z: 433.3 [MH⁺]

### Example 32

### N-(2-{3-[3-(4-Chloro-phenoxy)-pyrrolidin-1-yl]-2-hydroxy-propoxy}-phenyl)-3-methyl-butyramide

APCI-MS m/z: 447.3 [MH⁺]

### Example 33

### N-(2-{3-[3-(4-Chloro-phenoxy)-pyrrolidin-1-yl]-2-hydroxy-propoxy}-phenyl)-2-methoxy-acetamide

APCI-MS m/z: 435.2 [MH⁺]

### Example 34

### N-(2-{3-[3-(4-Chloro-phenoxy)-pyrrolidin-1-yl]-2-hydroxy-propoxy}-phenyl)-2,2-dimethyl-propionamide

APCI-MS m/z: 447.2 [MH⁺]

### Example 35

### 5-Oxo-hexanoic acid (2-{3-[3-(4-chloro-phenoxy)-pyrrolidin-1-yl]-2-hydroxy-propoxy}-phenyl)-amide

APCI-MS m/z: 475.3 [MH⁺]

### Example 36

### Hexanoic acid (2-{3-[3-(4-chloro-phenoxy)-pyrrolidin-1-yl]-2-hydroxy-propoxy}-phenyl)-amide

APCI-MS m/z: 461.3 [MH⁺]

### Example 37

### 2-Chloro-N-(2-{3-[3-(4-chloro-phenoxy)-pyrrolidin-1-yl]-2-hydroxy-propoxy}-phenyl)-benzamide

APCI-MS m/z: 501.2 , 503.2 [MH⁺]

### Example 38

### 3-Chloro-N-(2-{3-[3-(4-chloro-phenoxy)-pyrrolidin-1-yl]-2-hydroxy-propoxy}-phenyl)-benzamide

APCI-MS m/z: 501.2 , 503.2 [MH⁺]

### Example 39

### (4R)-N-(2-{3-[3-(4-chlorophenoxy)-1-pyrrolidinyl]-2-hydroxypropoxy}phenyl)-1,3-thiazolidine-4-carboxamide ditrifluoroacetate

APCI-MS m/z: 478.2 [MH⁺]

### Example 40

### Thiophene-2-carboxylic acid (2-{3-[4-(3,4-dichloro-phenoxy)-piperidin-1-yl]-2-hydroxy-propoxy}-phenyl)-amide

APCI-MS m/z: 521.0 , 523.0 [MH⁺]

### Example 41

### N-(2-{3-[4-(3,4-Dichloro-phenoxy)-piperidin-1-yl]-2-hydroxy-propoxy}-phenyl)-benzamide

APCI-MS m/z: 515.2, 517.2[MH⁺]

### Example 42

### N-(2-{3-[4-(3,4-Dichloro-phenoxy)-piperidin-1-yl]-2-hydroxy-propoxy}-phenyl)-nicotinamide

APCI-MS m/z: 516.2 , 518.2 [MH⁺]

### Example 43

### Pyridine-2-carboxylic acid (2-{3-[4-(3,4-dichloro-phenoxy)-piperidin-1-yl]-2-hydroxy-propoxy}-phenyl)-amide

APCI-MS m/z: 516.2 , 518.2 [MH⁺]

### Example 44

### N-(2-{3-[4-(3,4-Dichloro-phenoxy)-piperidin-1-yl]-2-hydroxy-propoxy}-phenyl)-isonicotinamide

APCI-MS m/z: 516.2 , 518.2 [MH⁺l

### Example 45

### Cyclohexanecarboxylic acid (2-{3-[4-(3,4-dichloro-phenoxy)-piperidin-1-yl]-2-hydroxy-propoxy}-phenyl)-amide

APCI-MS m/z: 521.3 , 523.3 [MH⁺]

### Example 46

### N-(2-{3-[4-(3,4-Dichloro-phenoxy)-piperidin-1-yl]-2-hydroxy-propoxy}-phenyl)-3-hydroxy-butyramide

APCI-MS m/z: 497.2 , 499.3 [MH⁺]

### Example 47

### 5-Methyl-thiophene-2-carboxylic acid (2-{3-[4-(3,4-dichloro-phenoxy)-piperidin-1-yl]-2-hydroxy-propoxy}-phenyl)-amide

APCI-MS m/z: 535.2 , 537.2 [MH⁺]

### Example 48

### Cyclobutanecarboxylic acid (2-{3-[4-(3,4-dichloro-phenoxy)-piperidin-1-yl]-2-hydroxy-propoxy}-phenyl)-amide

APCI-MS m/z:493.3 , 495.2 [MH⁺]

### Example 49

### N-(2-{3-[4-(3,4-Dichloro-phenoxy)-piperidin-1-yl]-2-hydroxy-propoxy}-phenyl)-propionamide

APCI-MS m/z: 467.2 , 469.2 [MH⁺]

### Example 50

### Pentanoic acid (2-{3-[4-(3,4-dichloro-phenoxy)-piperidin-1-yl]-2-hydroxy-propoxy}-phenyl)-amide

APCI-MS m/z: 495.3 , 497.3 [MH⁺]

### Example 51

### Pent-4-enoic acid (2-{3-[4-(3,4-dichloro-phenoxy)-piperidin-1-yl]-2-hydroxy-propoxy}-phenyl)-amide

APCI-MS m/z: 493.3 ,495.2 [MH⁺]

### Example 52

### Cyclopentanecarboxylic acid (2-{3-[4-(3,4-dichloro-phenoxy)-piperidin-1-yl]-2-hydroxy-propoxy}-phenyl)-amide

APCI-MS m/z: 507.3 , 509.3 [MH⁺]

### Example 53

### N-(2-{3-[4-(3,4-Dichloro-phenoxy)-piperidin-1-yl]-2-hydroxy-propoxy}-phenyl)-3-methyl-butyramide

APCI-MS m/z: 495.3,497.3 [MH⁺]

### Example 54

### N-(2-{3-[3-(4-chlorophenoxy)-1-pyrrolidinyl]-2-hydroxypropoxy}phenyl)-2,2,2-trifluoroacetamide hydrochloride

A mixture of 1-(2-aminophenoxy)-3-[3-(4-chlorophenoxy)-1-pyrrolidinyl]-2-propanol (10mg, 0.022mmol), dichloromethane (3ml) and Triethyl amine was cooled in an ice bath. A solution of Trifluoro acetic anhydride (3.5µl, 0.025mmol) in dichloromethane (2ml) was then added and the mixture stirred at 0°C until reaction completion. The mixture was diluted with dichloromethane, washed with 1M H₂SO₄, water, dried over natrium sulphate and concentrated to give an oil. The oil was treated with 1.0M ethereal HCl solution to give the product as solid (9mg).

APCI-MS: m/z 459, 460 [MH⁺]

### Example 55

### 4-(2-{3-[4-(3,4-Dichloro-phenoxy)-piperidin-1-yl]-2-hydroxy-propoxy}-phenylcarbamoyl)-3-methyl-butyric acid

1-(2-aminophenoxy)-3-[4-(3,4-dichlorophenoxy)-1-piperidinyl]-2-propanol (75uL, 0.2M/NMP) was mixed with 3-methyl glutaric anhydride (3eq, 225uL 0.2M /NMP) to get a product containing both esther and amide. After evaporation of the mixture it was treated with 3 eq 0.5M LiOH in (THF/water 1:4) for two hours at 80°C to hydrolyse the esther. The reaction mixture was diluted with more water (2mL) and the desired product was extracted with 5x500uL EtOAc which was evaporated to dryness.

APCI-MS m/z: 539.2 , 541.2 [MH⁺]

### Example 56

### N-(2-{3-[4-(3,4-Dichloro-phenoxy)-piperidin-1-yl]-2-hydroxy-propoxy}-phenyl)-succinamic acid

Prepared according to the method described in Example 55.

### APCI-MS m/z: 511.2 , 513.2 [MH⁺]

### Aniline Intermediate 3

### 1-(2-amino-5-methylphenoxy)-3-[3-(4-chlorophenoxy)-1-pyrrolidinyl]-2-propanol

APCI-MS m/z: 377.2 , 379.1 [MH+]
¹H NMR (400 MHz, CDCl3) : δ 7.26-7.21 (m, 2H), 6.79-6.74 (m, 2H), 6.67-6.62 (m, 3H), 4.83-4.76 (m, 1H), 4.15-4.06 (m, 1H), 4.04-4.00 (d, 2H), 3.73-3.64 (s, 2H), 3.47-3.35 (s, 1H), 3.14 -2.56 (m, 6H), 2.36-2.22(m, 4H), 2.05-1.95(m, 1H)

### Aniline Intermediate 4

### 1-(2-amino-5-methylphenoxy)-3-[3-(4-fluorophenoxy)-1-pyrrolidinyl]-2-propanol

APCI-MS m/z: 361.1 [MH+]
¹H NMR (400 MHz, CDCl3) : δ 7.00-6.94 (m, 2H), 6.81-6.76 (m, 2H), 6.67-6.62 (m, 3H), 4.81-4.74 (m, 1H), 4.15-4.06 (m, 1H), 4.03-3.99 (m, 2H), 3.88-3.36 (m, 3H), 3.12 -2.56 (m, 6H), 2.33-2.23(m, 4H), 2.05-1.96(m, 1H)

The compounds of Examples 57 to 85 were prepared using one of the Aniline Intermediates 3 and 4.

### Example 57

### Furan-2-carboxylic acid (2-{3-[3-(4-chloro-phenoxy)-pyrrolidin-1-yl]-2-hydroxy-propoxy}-4-methyl-phenyl)-amide

APCI-MS m/z: 471.5 , 473.5 [MH+]

### Example 58

### 1H-Pyrrole-2-carboxylic acid (2-{3-[3-(4-chloro-phenoxy)-pyrrolidin-1-yl]-2-hydroxy-propoxy}-4-methyl-phenyl)-amide

APCI-MS m/z: 470.5 , 472.5 [MH+]

### Example 59

### Thiophene-2-carboxylic acid (2-{3-[3-(4-chloro-phenoxy)-pyrrolidin-1-yl]-2-hydroxy-propoxy}-4-methyl-phenyl)-amide

APCI-MS m/z: 487.5 , 489.5 [MH+]

### Example 60

### Cyclopentanecarboxylic acid (2-{3-[3-(4-chloro-phenoxy)-pyrrolidin-1-yl]-2-hydroxy-propoxy}-4-methyl-phenyl)-amide

APCI-MS m/z: 473.6 , 475.5 [MH+]

### Example 61

### 5-Methyl-thiophene-2-carboxylic acid (2-{3-[3-(4-chloro-phenoxy)-pyrrolidin-1-yl]-2-hydroxy-propoxy}-4-methyl-phenyl)-amide

APCI-MS m/z: 501.5 , 503.5 [MH+]

### Example 62

### 3-Chloro-thiophene-2-carboxylic acid (2-{3-[3-(4-chloro-phenoxy)-pyrrolidin-1-yl]-2-hydroxy-propoxy}-4-methyl-phenyl)-amide

APCI-MS m/z: 521.5 , 532.5 [MH+]

### Example 63

### 5-Methyl-isoxazole-4-carboxylic acid (2-{3-[3-(4-chloro-phenoxy)-pyrrolidin-1-yl]-2-hydroxy-propoxy}-4-methyl-phenyl)-amide

APCI-MS m/z: 486.5 , 488.6 [MH+]

### Example 64

### [1,2,3]Thiadiazole-4-carboxylic acid (2-{3-[3-(4-chloro-phenoxy)-pyrrolidin-1-yl]-2-hydroxy-propoxy}-4-methyl-phenyl)-amide

APCI-MS m/z: 489.5 , 491.5[MH+]

### Example 65

### 3-Methyl-furan-2-carboxylic acid (2-{3-[3-(4-chloro-phenoxy)-pyrrolidin-1-yl]-2-hydroxy-propoxy}-4-methyl-phenyl)-amide

APCI-MS m/z: 485.5 , 487.6 [MH+]

### Example 66

### Cyclopent-1-enecarboxylic acid (2-{3-[3-(4-chloro-phenoxy)-pyrrolidin-1-yl]-2-hydroxy-propoxy}-4-methyl-phenyl)-amide

APCI-MS m/z: 471.6,473.6 [MH+]

### Example 67

### 2-Methyl-furan-3-carboxylic acid (2-{3-[3-(4-chloro-phenoxy)-pyrrolidin-1-yl]-2-hydroxy-propoxy}-4-methyl-phenyl)-amide

APCI-MS m/z: 485.6 , 487.6 [MH+]

### Example 68

### 3-Methyl-thiophene-2-carboxylic acid (2-{3-[3-(4-chloro-phenoxy)-pyrrolidin-1-yl]-2-hydroxy-propoxy}-4-methyl-phenyl)-amide

APCI-MS m/z: 501.6, 503.5 [MH+]

### Example 69

### 5-Nitro-1H-pyrazole-3-carboxylic acid (2-{3-[3-(4-chloro-phenoxy)-pyrrolidin-1-yl]-2-hydroxy-propoxy}-4-methyl-phenyl)-amide

APCI-MS m/z: 516.5 , 518.5 [MH+]

### Example 70

### Thiophene-3-carboxylic acid (2-{3-[3-(4-chloro-phenoxy)-pyrrolidin-1-yl]-2-hydroxy-propoxy}-4-methyl-phenyl)-amide

APCI-MS m/z: 487.5 , 489.5 [MH+]

### Example 71

### Cyclobutanecarboxylic acid (2-{3-[3-(4-chloro-phenoxy)-pyrrolidin-1-yl]-2-hydroxy-propoxy}-4-methyl-phenyl)-amide

APCI-MS m/z: 459.5 , 461.5 [MH+]

### Example 72

### Furan-2-carboxylic acid (2-{3-[3-(4-fluoro-phenoxy)-pyrrolidin-1-yl]-2-hydroxy-propoxy)-4-methyl-phenyl)-amide

APCI-MS m/z: 455.5 [MH+]

### Example 73

### 1H-Pyrrole-2-carboxylic acid (2-{3-[3-(4-fluoro-phenoxy)-pyrrolidin-1-yl]-2-hydroxy-propoxy}-4-methyl-phenyl)-amide

APCI-MS m/z: 454.6 [MH+]

### Example 74

### Thiophene-2-carboxylic acid (2-{3-[3-(4-fluoro-phenoxy)-pyrrolidin-1-yl]-2-hydroxy-propoxy}-4-methyl-phenyl)-amide

APCI-MS m/z: 471.5 [MH+]

### Example 75

### 3-Chloro-thiophene-2-carboxylic acid (2-{3-[3-(4-fluoro-phenoxy)-pyrrolidin-1-yl]-2-hydroxy-propoxy}-4-methyl-phenyl)-amide

APCI-MS m/z: 505.5 , 507.5 [MH+]

### Example 76

### 5-Methyl-isoxazole-4-carboxylic acid (2-{3-[3-(4-fluoro-phenoxy)-pyrrolidin-1-yl]-2-hydroxy-propoxy}-4-methyl-phenyl)-amide

APCI-MS m/z: 470.5 [MH+]

### Example 77

### 3-Methyl-furan-2-carboxylic acid (2-{3-[3-(4-fluoro-phenoxy)-pyrrolidin-1-yl]-2-hydroxy-propoxy}-4-methyl-phenyl)-amide

APCI-MS m/z: 469.6 [MH+]

### Example 78

### Cyclopent-1-enecarboxylic acid (2-{3-[3-(4-fluoro-phenoxy)-pyrrolidin-1-yl]-2-hydroxy-propoxy}-4-methyl-phenyl)-amide

APCI-MS m/z: 455.6 [MH+]

### Example 79

### 2-Methyl-furan-3-carboxylic acid (2-{3-[3-(4-fluoro-phenoxy)-pyrrolidin-1-yl]-2-hydroxy-propoxy}-4-methyl-phenyl)-amide

APCI-MS m/z: 469.6 [MH+]

### Example 80

### 3-Methyl-thiophene-2-carboxylic acid (2-{3-[3-(4-fluoro-phenoxy)-pyrrolidin-1-yl]-2-hydroxy-propoxy}-4-methyl-phenyl)-amide

APCI-MS m/z: 485.5 [MH+]

### Example 81

### 5-Chloro-thiophene-2-carboxylic acid (2-{3-[3-(4-fluoro-phenoxy)-pyrrolidin-1-yl]-2-hydroxy-propoxy}-4-methyl-phenyl)-amide

APCI-MS m/z: 505.5 , 507.5 [MH+]

### Example 82

### Thiophene-3-carboxylic acid (2-{3-[3-(4-fluoro-phenoxy)-pyrrolidin-1-yl]-2-hydroxy-propoxy}-4-methyl-phenyl)-amide

APCI-MS m/z: 471.5 [MH+]

### Example 83

### 2,5-Dimethyl-furan-3-carboxylic acid (2-{3-[3-(4-fluoro-phenoxy)-pyrrolidin-1-yl]-2-hydroxy-propoxy}-4-methyl-phenyl)-amide

APCI-MS m/z: 483.6 [MH+]

### Example 84

### Cyclobutanecarboxylic acid (2-{3-[3-(4-fluoro-phenoxy)-pyrrolidin-1-yl]-2-hydroxy-propoxy}-4-methyl-phenyl)-amide

APCI-MS m/z: 443.6 [MH+]

### Example 85

### Furan-3-carboxylic acid (2-{3-[3-(4-fluoro-phenoxy)-pyrrolidin-1-yl]-2-hydroxy-propoxy}-4-methyl-phenyl)-amide

APCI-MS m/z: 455.5 [MH+]

### Example 86

### N-{2-[(3-{3-[(4-fluorophenyl)oxy]-1-pyrrolidinyl}-2-hydroxypropyl)oxy]-4-methylphenyl}-1H-pyrrole-2-carboxamide

APCI-MS: m/z 454.1 [M+H⁺]

### Example 87

### N-{2-[(3-{3-[(4-chlorophenyl)oxy]-1-pyrrolidinyl}-2-hydroxypropyl)oxy]-4-methylphenyl}-3-thiophenecarboxamide

APCI-MS: m/z 471.1 [M+H⁺]

### Example 88

### N-{2-[(3-{3-[(4-chlorophenyl)oxy]-1-pyrrolidinyl}-2-hydroxy-2-methylpropyl)oxy]phenyl}-2-thiophenecarboxamide, compound with trifluoroacetic acid

Aniline intermediate 3 (60 mg, 0.159 mmol), 2-thiophenecarboxylic acid (20.4 mg, 0.159 mmol) and HATU (72 mg, 0.191 mmol) were stirred in dichloromethane (2 ml).

Diisopropylethylamine was added to pH 8. The mixture was stirred overnight and then concentrated. The residue was purified on silica (dichloromethane/methanol 98/2) followed by purification on C18 (2 g Isolute, acetonitrile/water 20/80 to 35/65 with 0.5% trifluoroacetic acid) to give the title compound (75 mg, 79%).

¹H-NMR (400 MHz, MeOD): δ 7.86 (m, 1H), 7.72 (m, 1H), 7.50 (m, 1H), 7.29 (m, 3H), 7.16 (m, 2H), 7.07 (m, 1H), 6.91 (m, 2H), 5.10 (m, 1H), 3.82-4.17 (m, 4H), 3.24-3.69 (m, 4H), 2.13-2.64 (m, 2H), 1.38 (m, 3H).

MS-APCI+: m/z 487 [MH⁺]

### Example 89

### N-{2-[(3-{3-[(4-fluorophenyl)oxy]-1-pyrrolidinyl}-2-hydroxypropyl)oxy]-4-methylphenyl}-2-thiophenecarboxamide

APCI MS APCI-MS: m/z 471.1 [M+H⁺]

### Example 90

### N-{2-[(3-{3-[(4-chlorophenyl)oxy]-1-pyrrolidinyl}-2-hydroxypropyl)oxy]phenyl}-2-furancarboxamide

APCI-MS: m/z 456.9 [M+H⁺]

### Example 91

### N-{2-[(3-{3-[(4-chlorophenyl)oxy]-1-pyrrolidinyl}-2-hydroxypropyl)oxy]phenyl}-1-pyrrole-2-carboxamide

APCI-MS: m/z 456.1 [M+H⁺]

### Example 92

### N-{2-[(3-{3-[(4-chlorophenyl)oxy]-1-pyrrolidinyl}-2-hydroxypropyl)oxy]-4-methylphenyl}-1H-pyrrole-3-carboxamide

APCI-MS: m/z 470.0 [M+H⁺]

### Example 93

### N-{2-[(3-{3-[(4-fluorophenyl)oxy]-1-pyrrolidinyl}-2-hydroxypropyl)oxy]-4-methylphenyl}-2-furancarboxamide

APCI-MS: m/z 455.1 [M+H⁺]

### Example 94

### N-{2-[(3-{3-[(4-chlorophenyl)oxy]-1-pyrrolidinyl}-2-hydroxy-2-methylpropyl)oxy]phenyl}cyclopentanecarboxamide, compound witt trifluoracetic acid

The compound (80 mg, 86%) was prepared from aniline intermediate 3 (60 mg, 0.159 mmol) and cyclopentanecarboxylic acid (18 µl, 0.159 mmol) as described in Example 88.

¹H-NMR (400 MHz, MeOD): δ 7.59 (m, 1H), 7.29 (m, 2H), 7.19 (m, 1H), 7.09 (m, 1H), 6.97 (m, 3H), 5.17 (m, 1H), 3.86-4.23 (m, 4H), 3.35-3.73 (m, 4H), 2.86 (m, 1H), 1.45 (bs, 3H).

MS-APCI+: m/z 473 [MH⁺]

### Example 95

### N-(2-{3-[3-(4-Fluoro-phenoxy)-pyrrolidin-1-yl]-2-hydroxy-2-methyl-propoxy}-phenyl)-benzamide

The compound was prepared using an analogous method as in Example 88.

APCI-MS: m/z 465 [MH⁺]

### Example 96

### N-(2-{3-[3-(4-Cyano-phenoxy)-pyrrolidin-1-yl]-2-hydroxy-2-methyl-propoxy}-phenyl)-benzamide

The compound was prepared using an analogous method as in Example 88.

APCI-MS: m/z 472 [MH⁺]

### Example 97

### N-(2-{3-[4-(3,4-Dichloro-phenoxy)-piperidin-1-yl]-2-hydroxy-2-methyl-propoxy}-phenyl)-benzamide

The compound was prepared using an analogous method as in Example 88.

APCI-MS: m/z 529 [MH⁺]

### Example 98

### N-(2-{3-[3-(4-Chloro-phenoxy)-pyrrolidin-1-yl]-2-hydroxy-2-methyl-propoxy}-phenyl)-benzamide

The compound was prepared using an analogous method as in Example 88.

APCI-MS: m/z 481 [MH⁺]

### Example 99

### N-(2-{3-[4-(3,4-Dichloro-phenylamino)-piperidin-1-yl]-2-hydroxy-2-methyl-propoxy}-phenyl)-benzamide

The compound was prepared using an analogous method as in Example 88.

### APCI-MS: m/z 528 [MH⁺]

### THP-1 Chemotaxis Assay

### Introduction

The assay measured the chemotactic response elicited by MIP-1α chemokine in the human monocytic cell line THP-1. The compounds of the Examples were evaluated by their ability to depress the chemotactic response to a standard concentration of MIP-1α chemokine.

### Methods

### Culture of THP-1 cells

Cells were thawed rapidly at 37°C from frozen aliquots and resuspended in a 25 cm flask containing 5 ml of RPMI-1640 medium supplemented with Glutamax and 10% heat inactivated fetal calf serum without antibiotics (RPMI+10%HIFCS). At day 3 the medium is discarded and replaced with fresh medium.

THP-1 cells are routinely cultured in RPMI-1640 medium supplemented with 10% heat inactivated fetal calf serum and glutamax but without antibiotics. Optimal growth of the cells requires that they are passaged every 3 days and that the minimum subculture density is 4x10+5 cells/ml.

### Chemotaxis assay

Cells were removed from the flask and washed by centrifugation in RPMI+10%HIFCS+glutamax. The cells were then resuspended at 2x10+7 cells/ml in fresh medium (RPMI+10%HIFCS+glutamax) to which was added calcein-AM (5 µl of stock solution to 1 ml to give a final concentration of 5x10⁻⁶M). After gentle mixing the cells were incubated at 37°C in a CO₂ incubator for 30 minutes. The cells were then diluted to 50 ml with medium and washed twice by centrifugation at 400xg. Labelled cells were then resuspended at a cell concentration of 1x10+7 cells/ml and incubated with an equal volume of MIP-1α antagonist (10⁻¹⁰M to 10⁻⁶M final concentration) for 30 minutes at 37°C in a humidified CO₂ incubator.

Chemotaxis was performed using Neuroprobe 96-well chemotaxis plates employing 8 µm filters (cat no. 101-8). Thirty microlitres of chemoattractant supplemented with various concentrations of antagonists or vehicle were added to the lower wells of the plate in triplicate. The filter was then carefully positioned on top and then 25µl of cells preincubated with the corresponding concentration of antagonist or vehicle were added to the surface of the filter. The plate was then incubated for 2 hours at 37°C in a humidified CO₂ incubator. The cells remaining on the surface were then removed by adsorption and the whole plate was centrifuged at 2000 rpm for 10 minutes. The filter was then removed and the cells that had migrated to the lower wells were quantified by the fluorescence of cell associated calcein-AM. Cell migration was then expressed in fluorescence units after subtraction of the reagent blank and values were standardized to % migration by comparing the fluorescence values with that of a known number of labelled cells. The effect of antagonists was calculated as % inhibition when the number of migrated cells were compared with vehicle.

## Claims

1. A compound of general formula 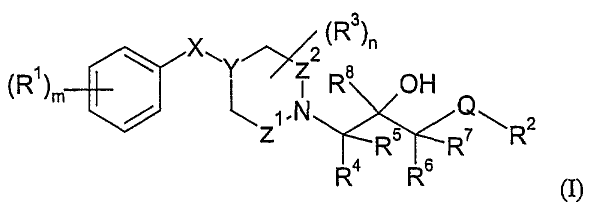 wherein:
m is 0, 1, 2 or 3;
each R¹ independently represents halogen, cyano, nitro, carboxyl, hydroxyl, C₃-C₆ cycloalkyl, C₁-C₆ alkoxy, C₁-C₆ alkoxycarbonyl, C₁-C₆ haloalkyl, C₁-C₆ haloalkoxy, -NR⁹R¹⁰, C₃-C₆ cycloalkylamino, C₁-C₆ alkylthio, C₁-C₆ alkylcarbonyl, C₁-C₆ alkylcarbonylamino, sulphonamido, C₁-C₆ alkylsulphonyl, -C(O)NR¹¹R¹², -NR¹³C(O)-(NH)ₚR¹⁴, phenyl, or C₁-C₆ alkyl optionally substituted by carboxyl or C₁-C₆ alkoxycarbonyl;
p is 0 or 1;
X represents an oxygen atom or a CH₂, OCH₂, CH₂O, CH₂NH, NH, carbonyl or sulphonyl group and Y represents a nitrogen atom or a CH or C(OH) group, provided that when X represents an oxygen atom or a CH₂O, CH₂NH or NH group, then Y represents a CH group;
Z¹ represents a bond or a group (CH₂)_{q} where q is 1 or 2;
Z² represents a bond or a group CH₂, with the proviso that Z¹ and Z² do not both simultaneously represent a bond;
Q represents an oxygen or sulphur atom or a group CH₂ or NH;
R² represents a group 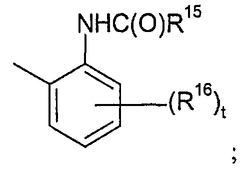
n is 0, 1 or 2;
each R³ independently represents a C₁-C₆ alkyl, C₁-C₆ alkoxycarbonyl, -CH₂OH or carboxyl group;
R⁴, R⁵, R⁶ and R⁷ each independently represent a hydrogen atom or a C₁-C₆ alkyl group, or R⁴, R⁵, R⁶ and R⁷ together represent a C₁-C₄ alkylene chain linking the two carbon atoms to which they are attached to form a 4- to 7-membered saturated carbocycle, or R⁵, R⁶ and R⁷ each represent a hydrogen atom and R⁴ and R⁸ together with the carbon atoms to which they are attached form a 5- to 6-membered saturated carbocycle;
R⁸ represents a hydrogen atom, a C₁-C₆ alkyl group or is linked to R⁴ as defined above;
R⁹ and R¹⁰ each independently represent a hydrogen atom or a C₁-C₆ alkyl group, or R⁹ and R¹⁰ together with the nitrogen atom to which they are attached form a 4- to 7-membered saturated heterocycle;
R¹¹ and R¹² each independently represent a hydrogen atom or a C₁-C₆ alkyl group optionally substituted by C₁-C₆ alkoxycarbonyl;
R¹³ represents a hydrogen atom or a C₁-C₆ alkyl group;
R¹⁴ represents a hydrogen atom, or a C₁-C₆ alkyl group optionally substituted by carboxyl, C₁-C₆ alkoxy or C₁-C₆ alkoxycarbonyl;
R¹⁵ represents a group C₂-C₆ alkyl, C₂-C₆ alkenyl, C₃-C₆ cycloalkyl, C₅-C₆ cycloalkenyl, adamantyl, phenyl or a saturated or unsaturated 5- to 10-membered heterocyclic ring system comprising at least one heteroatom selected from nitrogen, oxygen and sulphur, wherein each group may be optionally substituted by one or more substituents independently selected from nitro, hydroxyl, oxo, halogen, carboxyl, C₁-C₆ alkyl, C₁-C₆ alkoxy, C₁-C₆ alkylthio, C₁-C₆ alkylcarbonyl, C₁-C₆ alkoxycarbonyl, phenyl and -NHC(O)-R¹⁷, with the proviso that R¹⁵ does not represent an unsubstituted 1-pyrrolidinyl, an unsubstituted 1-piperidinyl or an unsubstituted 1-hexamethyleneiminyl group;
t is 0, 1, 2 or 3;
each R¹⁶ independently represents halogen, cyano, nitro, carboxyl, hydroxyl, C₃-C₆ cycloalkyl, C₁-C₆ alkoxy, C₁-C₆ alkoxycarbonyl, C₁-C₆ haloalkyl, C₁-C₆ haloalkoxy, -NR¹⁸R¹⁹, C₃-C₆ cycloalkylamino, C₁-C₆ alkylthio, C₁-C₆ alkylcarbonyl, C₁-C₆ alkylcarbonylamino, sulphonamido (-SO₂NH₂), C₁-C₆ alkylsulphonyl, -C(O)NR²⁰R²¹, -NR²²C(O)(NH)ᵥR²³, phenyl, or C₁-C₆ alkyl optionally substituted by carboxyl or C₁-C₆ alkoxycarbonyl;
R¹⁷ represents a C₁-C₆ alkyl, amino or phenyl group;
R¹⁸ and R¹⁹ each independently represent a hydrogen atom or a C₁-C₆ alkyl group, or R¹⁸ and R¹⁹ together with the nitrogen atom to which they are attached form a 4- to 7-membered saturated heterocycle;
R²⁰ and R²¹ each independently represent a hydrogen atom or a C₁-C₆ alkyl group optionally substituted by C₁-C₆ alkoxycarbonyl;
v is 0 or 1;
R²² represents a hydrogen atom or a C₁-C₆ alkyl group; and
R²³ represents a hydrogen atom, or a C₁-C₆ alkyl group optionally substituted by carboxyl, C₁-C₆ alkoxy or C₁-C₆ alkoxycarbonyl;
or a pharmaceutically acceptable salt or solvate thereof.

2. A compound according to claim 1, wherein X represents an oxygen atom or a CH₂ or NH group.

3. A compound according to claim 1, wherein Y represents a CH group.

4. A compound according to any one of claims 1 to 3, wherein Q represents an oxygen atom.

5. A compound according to any one of claims 1 to 4, wherein R¹⁵ represents a group C₂-C₅ alkyl, C₂-C₄ alkenyl, C₃-C₆ cycloalkyl, C₅-C₆ cycloalkenyl, adamantyl, phenyl or a saturated or unsaturated 5- to 10-membered heterocyclic ring system comprising at least one heteroatom selected from nitrogen, oxygen and sulphur, wherein each group may be optionally substituted by one, two or three substituents independently selected from hydroxyl, oxo, halogen, carboxyl, C₁-C₆ alkyl, C₁-C₆ alkoxy, C₁-C₆ alkylthio, C₁-C₆ alkylcarbonyl, C₁-C₆ alkoxycarbonyl, phenyl and -NHC(O)-R¹⁷.

6. A compound according to claim 5, wherein the saturated or unsaturated 5- to 10-membered heterocyclic ring system comprising at least one heteroatom selected from nitrogen, oxygen and sulphur, is pyrrolidinyl, piperidinyl, pyrazolyl, thiazolidinyl, thienyl, thiadiazolyl, isoxazolyl, pyrrolyl, furanyl, thiazolyl, indolyl, quinolinyl, benzimidazolyl, triazolyl, tetrazolyl or pyridinyl.

7. A compound according to any one of claims 1 to 6, wherein each R¹⁶ independently represents halogen, cyano, C₁-C₄ alkoxy, C₁-C₄ alkoxycarbonyl, C₁-C₄ haloalkyl, C₁-C₄ alkylcarbonyl, phenyl or C₁-C₄ alkyl.

8. A compound of formula (I), or a pharmaceutically acceptable salt or solvate thereof, as defined in claim 1 being selected from:
*N*-(5-Chloro-2-{3-[3-(4-chloro-phenoxy)-pyrrolidin-1-yl]-2-hydroxy-propoxy}-phenyl)-isobutyramide,
Thiophene-2-carboxylic acid (2-{3-[3-(4-chloro-phenoxy)-pyrrolidin-1-yl]-2-hydroxy-propoxy}-phenyl)-amide,
N-[(2-{3-[3-(4-Chloro-phenoxy)-pyrrolidin-1-yl]-2-hydroxy-propoxy}-phenylcarbamoyl)-methyl]-benzamide,
Pyrazine-2-carboxylic acid (2-{3-[3-(4-chloro-phenoxy)-pyrrolidin-1-yl]-2-hydroxy-propoxy}-phenyl)-amide,
Cyclohexanecarboxylic acid (2-{3-[3-(4-chloro-phenoxy)-pyrrolidin-1-yl]-2-hydroxy-propoxy}-phenyl)-amide,
N-(2-{3-[3-(4-Chloro-phenoxy)-pyrrolidin-1-yl]-2-hydroxy-propoxy}-phenyl)-phthalamic acid methyl ester,
N-(2-{3-[3-(4-Chloro-phenoxy)-pyrrolidin-1-yl]-2-hydroxy-propoxy}-phenyl)-3-hydroxy-butyramide,
N-(2-{3-[3-(4-Chloro-phenoxy)-pyrrolidin-1-yl]-2-hydroxy-propoxy}-phenyl)-2-ureido-acetamide,
4-Acetylamino-N-(2-{3-[3-(4-chloro-phenoxy)-pyrrolidin-1-yl]-2-hydroxy-propoxy}-phenyl)-butyramide,
1-Acetyl-piperidine-4-carboxylic acid (2-{3-[3-(4-chloro-phenoxy)-pyrrolidin-1-yl]-2-hydroxy-propoxy}-phenyl)-amide,
N-(2-{3-[3-(4-Chloro-phenoxy)-pyrrolidin-1-yl]-2-hydroxy-propoxy}-phenyl)-3-methoxy-benzamide,
2-Acetylamino-N-(2-{3-[3-(4-chloro-phenoxy)-pyrrolidin-1-yl]-2-hydroxy-propoxy}-phenyl)-3-methyl-butyramide,
2-Acetylamino-N-(2-{3-[3-(4-chloro-phenoxy)-pyrrolidin-1-yl]-2-hydroxy-propoxy}-phenyl)-3-hydroxy-butyramide,
Adamantane-1-carboxylic acid (2-{3-[3-(4-chloro-phenoxy)-pyrrolidin-1-yl]-2-hydroxy-propoxy}-phenyl)-amide,
2-Acetylamino-N-(2-{3-[3-(4-chloro-phenoxy)-pyrrolidin-1-yl]-2-hydroxy-propoxy}-phenyl)-3-phenyl-propionamide,
N-(2-{3-[3-(4-Chloro-phenoxy)-pyrrolidin-1-yl]-2-hydroxy-propoxy}-phenyl)-2-methoxy-benzamide,
5-Methyl-thiophene-2-carboxylic acid (2-{3-[3-(4-chloro-phenoxy)-pyrrolidin-1-yl]-2-hydroxy-propoxy}-phenyl)-amide,
1-Acetyl-pyrrolidine-2-carboxylic acid (2-{3-[3-(4-chloro-phenoxy)-pyrrolidin-1-yl]-2-hydroxy-propoxy}-phenyl)-amide,
1,5-Dimethyl-1H-pyrazole-3-carboxylic acid (2-{3-[3-(4-chloro-phenoxy)-pyrrolidin-1-yl]-2-hydroxy-propoxy}-phenyl)-amide,
5-Oxo-pyrrolidine-2-carboxylic acid (2-{3-[3-(4-chloro-phenoxy)-pyrrolidin-1-yl]-2-hydroxy-propoxy}-phenyl)-amide,
1H-Indole-6-carboxylic acid (2-{3-[3-(4-chloro-phenoxy)-pyrrolidin-1-yl]-2-hydroxy-propoxy}-phenyl)-amide,
Cyclobutanecarboxylic acid (2-{3-[3-(4-chloro-phenoxy)-pyrrolidin-1-yl]-2-hydroxy-propoxy}-phenyl)-amide,
N-(2-{3-[3-(4-Chloro-phenoxy)-pyrrolidin-1-yl]-2-hydroxy-propoxy}-phenyl)-propionamide,
Pentanoic acid (2-{3-[3-(4-chloro-phenoxy)-pyrrolidin-1-yl]-2-hydroxy-propoxy}-phenyl)-amide,
Pent-4-enoic acid (2-{3-[3-(4-chloro-phenoxy)-pyrrolidin-1-yl]-2-hydroxy-propoxy}-phenyl)-amide,
Cyclopentanecarboxylic acid (2-{3-[3-(4-chloro-phenoxy)-pyrrolidin-1-yl]-2-hydroxy-propoxy}-phenyl)-amide,
Cyclopropanecarboxylic acid (2-{3-[3-(4-chloro-phenoxy)-pyrrolidin-1-yl]-2-hydroxy-propoxy}-phenyl)-amide,
N-(2-{3-[3-(4-Chloro-phenoxy)-pyrrolidin-1-yl]-2-hydroxy-propoxy}-phenyl)-isobutyramide,
N-(2-{3-[3-(4-Chloro-phenoxy)-pyrrolidin-1-yl]-2-hydroxy-propoxy}-phenyl)-2-methylsulfanyl-acetamide,
2-Acetylamino-N-(2-{3-[3-(4-chloro-phenoxy)-pyrrolidin-1-yl]-2-hydroxy-propoxy}-phenyl)-propionamide,
N-(2-{3-[3-(4-Chloro-phenoxy)-pyrrolidin-1-yl]-2-hydroxy-propoxy}-phenyl)-butyramide,
N-(2-{3-[3-(4-Chloro-phenoxy)-pyrrolidin-1-yl]-2-hydroxy-propoxy}-phenyl)-3-methyl-butyramide,
N-(2-{3-[3-(4-Chloro-phenoxy)-pyrrolidin-1-yl]-2-hydroxy-propoxy}-phenyl)-2-methoxy-acetamide,
N-(2-{3-[3-(4-Chloro-phenoxy)-pyrrolidin-1-yl]-2-hydroxy-propoxy}-phenyl)-2,2-dimethyl-propionamide,
5-Oxo-hexanoic acid (2-{3-[3-(4-chloro-phenoxy)-pyrrolidin-1-yl]-2-hydroxy-propoxy}-phenyl)-amide,
Hexanoic acid (2-{3-[3-(4-chloro-phenoxy)-pyrrolidin-1-yl]-2-hydroxy-propoxy}-phenyl)-amide,
2-Chloro-N-(2-{3-[3-(4-chloro-phenoxy)-pyrrolidin-1-yl]-2-hydroxy-propoxy}-phenyl)-benzamide,
3-Chloro-N-(2-{3-[3-(4-chloro-phenoxy)-pyrrolidin-1-yl]-2-hydroxy-propoxy}-phenyl)-benzamide,
(4*R*)-*N*-(2-{3-[3-(4-chlorophenoxy)-1-pyrrolidinyl]-2-hydroxypropoxy}phenyl)-1,3-thiazolidine-4-carboxamide ditrifluoroacetate,
Thiophene-2-carboxylic acid (2-{3-[4-(3,4-dichloro-phenoxy)-piperidin-1-yl]-2-hydroxy-propoxy} -phenyl)-amide,
N-(2-{3-[4-(3,4-Dichloro-phenoxy)-piperidin-1-yl]-2-hydroxy-propoxy}-phenyl)-benzamide,
N-(2-{3-[4-(3,4-Dichloro-phenoxy)-piperidin-1-yl]-2-hydroxy-propoxy}-phenyl)-nicotinamide,
Pyridine-2-carboxylic acid (2-{3-[4-(3,4-dichloro-phenoxy)-piperidin-1-yl]-2-hydroxy-propoxy}-phenyl)-amide,
N-(2-{3-[4-(3,4-Dichloro-phenoxy)-piperidin-1-yl]-2-hydroxy-propoxy}-phenyl)-isonicotinamide,
Cyclohexanecarboxylic acid (2-{3-[4-(3,4-dichloro-phenoxy)-piperidin-1-yl]-2-hydroxy-propoxy}-phenyl)-amide,
N-(2-{3-[4-(3,4-Dichloro-phenoxy)-piperidin-1-yl]-2-hydroxy-propoxy}-phenyl)-3-hydroxy-butyramide,
5-Methyl-thiophene-2-carboxylic acid (2-{3-[4-(3,4-dichloro-phenoxy)-piperidin-1-yl]-2-hydroxy-propoxy}-phenyl)-amide,
Cyclobutanecarboxylic acid (2-{3-[4-(3,4-dichloro-phenoxy)-piperidin-1-yl]-2-hydroxy-propoxy}-phenyl)-amide,
N-(2-{3-[4-(3,4-Dichloro-phenoxy)-piperidin-1-yl]-2-hydroxy-propoxy}-phenyl)-propionamide,
Pentanoic acid (2-{3-[4-(3,4-dichloro-phenoxy)-piperidin-1-yl]-2-hydroxy-propoxy}-phenyl)-amide,
Pent-4-enoic acid (2-{3-[4-(3,4-dichloro-phenoxy)-piperidin-1-yl]-2-hydroxy-propoxy}-phenyl)-amide,
Cyclopentanecarboxylic acid (2-{3-[4-(3,4-dichloro-phenoxy)-piperidin-1-yl]-2-hydroxy-propoxy} -phenyl)-amide,
N-(2-{3-[4-(3,4-Dichloro-phenoxy)-piperidin-1-yl]-2-hydroxy-propoxy}-phenyl)-3-methyl-butyramide,
*N*-(2*-*{3-[3-(4-chlorophenoxy)-1-pyrrolidinyl]-2-hydroxypropoxy}phenyl)-2,2,2-trifluoroacetamide hydrochloride,
4-(2-{3-[4-(3,4-Dichloro-phenoxy)-piperidin-1-yl]-2-hydroxy-propoxy}-phenylcarbamoyl)-3-methyl-butyric acid,
N-(2-{3-[4-(3,4-Dichloro-phenoxy)-piperidin-1-yl]-2-hydroxy-propoxy}-phenyl)-succinamic acid,
Furan-2-carboxylic acid (2-{3-[3-(4-chloro-phenoxy)-pyrrolidin-1-yl]-2-hydroxy-propoxy}-4-methyl-phenyl)-amide,
1H-Pyrrole-2-carboxylic acid (2-{3-[3-(4-chloro-phenoxy)-pyrrolidin-1-yl]-2-hydroxy-propoxy}-4-methyl-phenyl)-amide,
Thiophene-2-carboxylic acid (2-{3-[3-(4-chloro-phenoxy)-pyrrolidin-1-yl]-2-hydroxy-propoxy}-4-methyl-phenyl)-amide,
Cyclopentanecarboxylic acid (2-{3-[3-(4-chloro-phenoxy)-pyrrolidin-1-yl]-2-hydroxy-propoxy}-4-methyl-phenyl)-amide,
5-Methyl-thiophene-2-carboxylic acid (2-{3-[3-(4-chloro-phenoxy)-pyrrolidin-1-yl]-2-hydroxy-propoxy}-4-methyl-phenyl)-amide,
3-Chloro-thiophene-2-carboxylic acid (2-{3-[3-(4-chloro-phenoxy)-pyrrolidin-1-yl]-2-hydroxy-propoxy}-4-methyl-phenyl)-amide,
5-Methyl-isoxazole-4-carboxylic acid (2-{3-[3-(4-chloro-phenoxy)-pyrrolidin-1-yl]-2-hydroxy-propoxy}-4-methyl-phenyl)-amide,
[1,2,3]Thiadiazole-4-carboxylic acid (2-{3-[3-(4-chloro-phenoxy)-pyrrolidin-1-yl]-2-hydroxy-propoxy}-4-methyl-phenyl)-amide,
3-Methyl-furan-2-carboxylic acid (2-{3-[3-{4-chloro-phenoxy)-pyrrolidin-1-yl]-2-hydroxy-propoxy}-4-methyl-phenyl)-amide,
Cyclopent-1-enecarboxylic acid (2-{3-[3-(4-chloro-phenoxy)-pyrrolidin-1-yl]-2-hydroxy-propoxy}-4-methyl-phenyl)-amide,
2-Methyl-furan-3-carboxylic acid (2-{3-[3-(4-chloro-phenoxy)-pyrrolidin-1-yl]-2-hydroxy-gropoxy}-4-methyl-phenyl)-amide,
3-Methyl-thiophene-2-carboxylic acid (2-{3-[3-(4-chloro-phenoxy)-pyrrolidin-1-yl]-2-hydroxy-propoxy}-4-methyl-phenyl)-amide,
5-Nitro-1H-pyrazole-3-carboxylic acid (2-{3-[3-(4-chloxo-phenoxy)-pyrrolidin-1-yl]-2-hydroxy-propoxy}-4-methyl-phenyl)-amide,
Thiophene-3-carboxylic acid (2-{3-[3-(4-chloro-phenoxy)-pyrrolidin-1-yl]-2-hydroxy-propoxy}-4-methyl-phenyl)-amide,
Cyclobutanecarboxylic acid (2-{3-[3-(4-chloro-phenoxy)-pyrrolidin-1-yl]-2-hydroxy-propoxy}-4-methyl-phenyl)-amide,
Furan-2-carboxylic acid (2-{3-[3-(4-fluoro-phenoxy)-pyrrolidin-1-yl]-2-hydroxy-propoxy}-4-methyl-phenyl)-amide,
1H-Pyrrole-2-carboxylic acid (2-{3-[3-(4-fluoro-phenoxy)-pyrrolidin-1-yl]-2-hydroxy-propoxy}-4-methyl-phenyl)-amide,
Thiophene-2-carboxylic acid (2-{3-[3-(4-fluoro-phenoxy)-pyrrolidin-1-yl]-2-hydroxy-propoxy}-4-methyl-phenyl)-amide,
3-Chloro-thiophene-2-carboxylic acid (2-{3-[3-(4-fluoro-phenoxy)-pyrrolidin-1-yl]-2-hydroxy-propoxy}-4-methyl-phenyl)-amide,
5-Methyl-isoxazole-4-carboxylic acid (2-{3-[3-(4-fluoro-phenoxy)-pyrrolidin-1-yl]-2-hydroxy-propoxy}-4-methyl-phenyl)-amide,
3-Methyl-furan-2-carboxylic acid (2-{3-[3-(4-fluoro-phenoxy)-pyrrolidin-1-yl]-2-hydroxy-propoxy}-4-methyl-phenyl)-amide,
Cyclopent-1-enecarboxylic acid (2-{3-[3-(4-fluoro-phenoxy)-pyrrolidin-1-yl]-2-hydroxy-propoxy}-4-methyl-phenyl)-amide,
2-Methyl-furan-3-carboxylic acid (2-{3-[3-(4-fluoro-phenoxy)-pyrrolidin-1-yl]-2-hydroxy-propoxy} -4-methyl-phenyl)-amide,
3-Methyl-thiophene-2-carboxylic acid (2-{3-[3-(4-fluoro-phenoxy)-pyrrolidin-1-yl]-2-hydroxy-propoxy}-4-methyl-phenyl)-amide,
5-Chloro-thiophene-2-carboxylic acid (2-{3-[3-(4-fluoro-phenoxy)-pyrrolidin-1-yl]-2-hydroxy-propoxy}-4-methyl-phenyl)-amide,
Thiophene-3-carboxylic acid (2-{3-[3-(4-fluoro-phenoxy)-pyrrolidin-1-yl]-2-hydroxy-propoxy}-4-methyl-phenyl)-amide,
2,5-Dimethyl-furan-3-carboxylic acid (2-{3-[3-(4-fluoro-phenoxy)-pyrrolidin-1-yl]-2-hydroxy-propoxy}-4-methyl-phenyl)-amide,
Cyclobutanecarboxylic acid (2-{3-[3-(4-fluoro-phenoxy)-pyrrolidin-1-yl]-2-hydroxy-propoxy}-4-methyl-phenyl)-amide,
Furan-3-carboxylic acid (2-{3-[3-(4-fluoro-phenoxy)-pyrrolidin-1-yl]-2-hydroxy-propoxy}-4-methyl-phenyl)-amide,
N-{2-[(3-{3-[(4-fluorophenyl)oxy]-1-pyrrolidinyl}-2-hydroxypropyl)oxy]-4-methylphenyl}-1H-pyrrole-2-carboxamide,
N-{2-[(3-{3-[(4-chlorophenyl)oxy]-1-pyrrolidinyl}-2-hydroxypropyl)oxy]-4-methylphenyl}-3-thiophenecarboxamide,
*N*-{2-[(3-{3-[(4-chlorophenyl)oxy]-1-pyrrolidinyl}-2-hydroxy-2-methylpropyl)oxy]phenyl}-2-thiophenecarboxamide, compound with trifluoroacetic acid,
N-{2-[(3-{3-[(4-fluorophenyl)oxy]-1-pyrrolidinyl}-2-hydroxypropyl)oxy]-4-methylphenyl}-2-thiophenecarboxamide,
N-{2-[(3-{3-[(4-chlorophenyl)oxy]-1-pyrrolidinyl}-2-hydroxypropyl)oxy]phenyl}-2-furancarboxamide,
N-{2-[(3-{3-[(4-chlorophenyl)oxy]-1-pyrrolidinyl}-2-hydroxypropyl)oxy]phenyl}-1-pyrrole-2-carboxamide
N-{2-[(3-{3-[(4-chlorophenyl)oxy]-1-pyrrolidinyl}-2-hydroxypropyl)oxy]-4-methylphenyl}-1H-pyrrole-3-carboxamide,
N-{2-[(3-{3-[(4-fluorophenyl)oxy]-1-pyrrolidinyl)-2-hydroxypropyl)oxy]-4-methylphenyl}-2-furancarboxamide,
N-{2-[(3-{3-[(4-chlorophenyl)oxy]-1-pyrrolidinyl}-2-hydroxy-2-methylpropyl)oxy]phenyl}cyclopentanecarboxamide, compound with trifluoracetic acid,
N-(2-{3-[3-(4-Fluoro-phenoxy)-pyrrolidin-1-yl]-2-hydroxy-2-methyl-propoxy}-phenyl)-benzamide,
N-(2-{3-[3-(4-Cyano-phenoxy)-pyrrolidin-1-yl]-2-hydroxy-2-methyl-propoxy}-phenyl)-benzamide,
N-(2-{3-[4-(3,4-Dichloro-phenoxy)-piperidin-1-yl]-2-hydroxy-2-methyl-propoxy}-phenyl)-benzamide,
N-(2-{3-[3-(4-Chloro-phenoxy)-pyrrolidin-1-yl]-2-hydroxy-2-methyl-propoxy}-phenyl)-benzamide, and
N-(2-{3-[4-(3,4-Dichloro-phenylamino)-piperidin-1-yl]-2-hydroxy-2-methylpropoxy}-phenyl)-benzamide.

9. A process for the preparation of a compound of formula (I) as defined in claim 1 which comprises reacting a compound of general formula 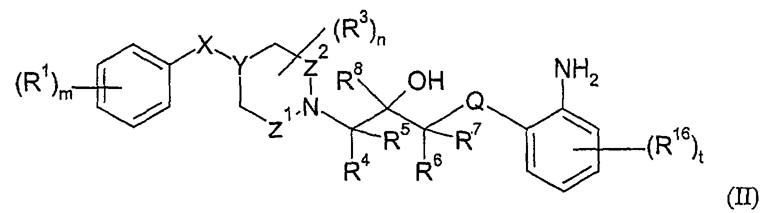 or a salt thereof, wherein m, n, t, R¹, R³, R⁴, R⁵, R⁶, R⁷, R⁸, R¹⁶, Q, Z¹ and Z² are as defined in formula (1), with a compound of general formula
R¹⁵ - CO₂H (III)
or chemically equivalent derivative thereof, wherein R¹⁵ is as defined in formula (I); and optionally thereafter forming a pharmaceutically acceptable salt or solvate of the compound of formula (I) obtained.

10. A pharmaceutical composition comprising a compound of formula (I), or a pharmaceutically acceptable salt or solvate thereof, as claimed in any one of claims 1 to 8 in association with a pharmaceutically acceptable adjuvant, diluent or carrier.

11. A process for the preparation of a pharmaceutical composition as claimed in claim 10 which comprises mixing a compound of formula (I), or a pharmaceutically acceptable salt or solvate thereof, as claimed in any one of claims 1 to 8 with a pharmaceutically acceptable adjuvant, diluent or carrier.

12. A compound of formula (I), or a pharmaceutically-acceptable salt or solvate thereof, as claimed in any one of claims 1 to 8 for use in therapy.

13. Use of a compound of formula (I), or a pharmaceutically acceptable salt or solvate thereof, as claimed in any one of claims 1 to 8 in the manufacture of a medicament for use in therapy.

14. Use of a compound of formula (I); or a pharmaceutically acceptable salt or solvate thereof, as claimed in any one of claims 1 to 8 in the manufacture of a medicament for the treatment of human diseases or conditions in which modulation of chemokine receptor activity is beneficial.

15. Use of a compound of formula (I), or a pharmaceutically acceptable salt or solvate thereof, as claimed in any one of claims 1 to 8 in the manufacture of a medicament for use in treating rheumatoid arthritis.

16. Use of a compound of formula (I), or a pharmaceutically acceptable salt or solvate thereof, as claimed in any one of claims 1 to 8 in the manufacture of a medicament for use in treating chronic obstructive pulmonary disease.

17. Use of a compound of formula (I), or a pharmaceutically acceptable salt or solvate thereof, as claimed in any one of claims 1 to 8 in the manufacture of a medicament for use in treating asthma.

18. Use of a compound of formula (I), or a pharmaceutically acceptable salt or solvate thereof, as claimed in any one of claims 1 to 8 in the manufacture of a medicament for use in treating multiple sclerosis.

## Patentansprüche

1. Verbindungen der allgemeinen Formel 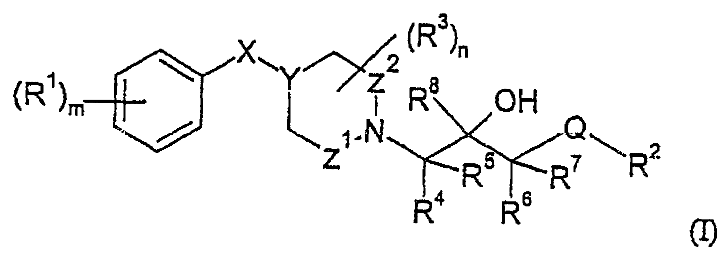 wobei:
m für 0, 1, 2 oder 3 steht;
die Reste R¹ jeweils unabhängig voneinander für Halogen, Cyano, Nitro, Carboxyl, Hydroxy, C₃-C₆-Cycloalkyl, C₁-C₆-Alkoxy, C₁-C₆-Alkoxycarbonyl, C₁-C₆-Halogenalkyl, C₁-C₆-Halogenalkoxy, -NR⁹R¹⁰, C₃-C₆-Cycloalkylamino, C₁-C₆-Alkylthio, C₁-C₆-Alkylcarbonyl, C₁-C₆-Akylcarbonylamino, Sulfonamido, C₁-C₆-Alkylsulfonyl, -C(O)NR¹¹R¹², -NR¹³C(O)-(NH)ₚR¹⁴, Phenyl oder gegebenenfalls durch Carboxyl oder C₁-C₆-Alkoxycarbonyl substituiertes C₁-C₆-Alkyl stehen;
p für 0 oder 1 steht;
X für ein Sauerstoffatom oder eine CH₂-, OCH₂-, CH₂O-, CH₂NH-, NH-, Carbonyl- oder Sulfonylgruppe steht und Y für ein Stickstoffatom oder eine CHoder C(OH)-Gruppe steht, mit der Maßgabe, daß, wenn X für ein Sauerstoffatom oder eine CH₂O-, CH₂NH- oder NH-Gruppe steht, Y für eine CH-Gruppe steht;
Z¹ für eine Bindung oder eine Gruppe (CH₂)_{q} steht, wobei q für 1 oder 2 steht;
Z² für eine Bindung oder eine Gruppe CH₂ steht, mit der Maßgabe, daß Z¹ und Z² nicht beide gleichzeitig für eine Bindung stehen;
Q für ein Sauerstoff- oder Schwefelatom oder eine Gruppe CH₂ oder NH steht;
R² für eine Gruppe 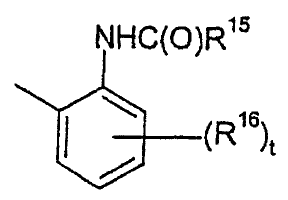 steht;
n für 0, 1 oder 2 steht;
die Reste R³ jeweils unabhängig voneinander für eine C₁-C₆-Alkyl-, C₁-C₆-Alkoxycarbonyl-, -CH₂OHoder Carboxylgruppe stehen;
R⁴, R⁵, R⁶ und R⁷ jeweils unabhängig voneinander für ein Wasserstoffatom oder eine C₁-C₆-Alkylgruppe stehen, oder R⁴, R⁵, R⁶ und R⁷ zusammen für eine C₁-C₄-Alkylkenkette stehen, die die beiden Kohlenstoffatome, an die sie gebunden sind, unter Bildung eines 4- bis 7gliedrigen gesättigten Carbocyclus verbinden, oder R⁵, R⁶ und R⁷ jeweils für ein Wasserstoffatom stehen und R⁴ und R⁸ zusammen mit den Kohlenstoffatomen, an die sie gebunden sind, einen 5- oder 6gliedrigen gesättigten Carbocyclus bilden;
R⁸ für ein Wasserstoffatom oder eine C₁-C₆-Alkylgruppe steht oder wie oben definiert mit R⁴ verbunden ist;
R⁹ und R¹⁰ jeweils unabhängig voneinander für ein Wasserstoffatom oder eine C₁-C₆-Alkylgruppe stehen, oder R⁹ und R¹⁰ zusammen mit dem Stickstoffatom, an das sie gebunden sind, einen 4- bis 7gliedrigen gesättigten Heterocyclus bilden;
R¹¹ und R¹² jeweils unabhängig voneinander für ein Wasserstoffatom oder eine C₁-C₆-Alkylgruppe, gegebenenfalls substituiert durch C₁-C₆-Alkoxycarbonyl, stehen;
R¹³ für ein Wasserstoffatom oder eine C₁-C₆-Alkylgruppe steht;
R¹⁴ für ein Wasserstoffatom oder eine C₁-C₆-Alkylgruppe, gegebenenfalls substituiert durch Carboxy, C₁-C₆-Alkoxy oder C₁-C₆-Alkoxycarbonyl, steht;
R¹⁵ für eine Gruppe C₂-C₆-Alkyl, C₂-C₆-Alkenyl, C₃-C₆-Cycloalkyl, C₅-C₆-Cycloalkenyl, Adamantyl, Phenyl oder ein gesättigtes oder ungesättigtes 5-bis 10gliedriges heterocyclisches Ringsystem enthaltend wenigstens ein Heteroatom ausgewählt aus Stickstoff, Sauerstoff und Schwefel, steht, wobei jede Gruppe gegebenenfalls durch einen oder mehrere Substituenten, unabhängig voneinander ausgewählt aus Nitro, Hydroxy, Oxo, Halogen, Carboxyl, C₁-C₆-Alkyl, C₁-C₆-Alkoxy, C₁-C₆-Alkylthio, C₁-C₆-Alkylcarbonyl, C₁-C₆-Alkoxycarbonyl, Phenyl und -NHC(O)-R¹⁷, substituiert sein kann, mit der Maßgabe, daß R¹⁵ nicht für eine unsubstituierte 1-Pyrrolidinyl-, eine unsubstituierte 1-Piperidinyl- oder eine unsubstituierte 1-Hexamethyleniminylgruppe steht;
t für 0, 1, 2 oder 3 steht;
die Reste R¹⁶ jeweils unabhängig voneinander für Wasserstoff, Cyano, Nitro, Carboxyl, Hydroxy, C₃-C₆-Cycloalkyl, C₁-C₆-Alkoxy, C₁-C₆-Alkoxycarbonyl, C₁-C₆-Halogenalkyl, C₁-C₆-Halogenalkoxy, -NR¹⁸R¹⁹, C₃-C₆-Cycloalkylamino, C₁-C₆-Alkylthio, C₁-C₆-Alkylcarbonyl, C₁-C₆-Akylcarbonylamino, Sulfonamido (-SO₂NH₂), C₁-C₆-Alkylsulfonyl, -C(O)NR²⁰R²¹, -NR²²C(O)-(NH)ᵥR²³, Phenyl oder gegebenenfalls durch Carboxyl oder C₁-C₆-Alkoxycarbonyl substituiertes C₁-C₆-Alkyl stehen;
R¹⁷ für eine C₁-C₆-Alkyl-, Amino- oder Phenylgruppe steht;
R¹⁸ und R¹⁹ jeweils unabhängig voneinander für ein Wasserstoffatom oder eine C₁-C₆-Alkylgruppe stehen oder R¹⁸ und R¹⁹ zusammen mit dem Stickstoffatom, an das sie gebunden sind, einen 4- bis 7gliedrigen gesättigten Heterocyclus bilden;
R²⁰ und R²¹ jeweils unabhängig voneinander für ein Wasserstoffatom oder eine C₁-C₆-Alkylgruppe, gegebenenfalls substituiert durch C₁-C₆-Alkoxycarbonyl, stehen;
v für 0 oder 1 steht;
R²² für ein Wasserstoffatom oder eine C₁-C₆-Alkylgruppe steht; und
R²³ für ein Wasserstoffatom oder eine C₁-C₆-Alkylgruppe, gegebenenfalls substituiert durch Carboxyl, C₁-C₆-Alkoxy oder C₁-C₆-Alkoxycarbonyl, steht;
und deren pharmazeutisch unbedenkliche Salze und Solvate.

2. Verbindungen nach Anspruch 1, wobei X für ein Sauerstoffatom oder eine CH₂- oder NH-Gruppe steht.

3. Verbindungen nach Anspruch 1, wobei Y für eine CH-Gruppe steht.

4. Verbindungen nach einem der Ansprüche 1 bis 3, wobei Q für ein Sauerstoffatom steht.

5. Verbindungen nach einem der Ansprüche 1 bis 4, wobei R¹⁵ für eine Gruppe C₂-C₅-Alkyl, C₂-C₄-Alkenyl, C₃-C₆-Cycloalkyl, C₅-C₆-Cycloalkenyl, Adamantyl, Phenyl oder ein gesättigtes oder ungesättigtes 5- bis 10gliedriges heterocyclisches Ringsystem, enthaltend wenigstens ein Heteroatom ausgewählt aus Stickstoff, Sauerstoff und Schwefel, steht, wobei jede Gruppe gegebenenfalls durch einen, zwei oder drei Substituenten, unabhängig voneinander ausgewählt aus Hydroxy, Oxo, Halogen, Carboxyl, C₁-C₆-Alkyl, C₁-C₆-Alkoxy, C₁-C₆-Alkylthio, C₁-C₆-Alkylcarbonyl, C₁-C₆-Alkoxycarbonyl, Phenyl und -NHC(O)-R¹⁷, substituiert sein kann.

6. Verbindungen nach Anspruch 5, wobei es sich bei dem gesättigten oder ungesättigten, 5- bis 10gliedrigen heterocyclischen Ringsystem, enthaltend wenigstens ein Heteroatom ausgewählt aus Stickstoff, Sauerstoff und Schwefel, um Pyrrolidinyl, Piperidinyl, Pyrazolyl, Thiazolidinyl, Thienyl, Thiadiazolyl, Isoxazolyl, Pyrrolyl, Furanyl, Thiazolyl, Indolyl, Chinolinyl, Benzimidazolyl, Triazolyl, Tetrazolyl oder Pyridinyl handelt.

7. Verbindungen nach einem der Ansprüche 1 bis 6, wobei die Reste R¹⁶ jeweils unabhängig voneinander für Halogen, Cyano, C₁-C₄-Alkoxy, C₁-C₄-Alkoxycarbonyl, C₁-C₄-Halogenalkyl, C₁-C₄-Alkylcarbonyl, Phenyl oder C₁-C₄-Alkyl stehen.

8. Verbindungen der Formel (I) und deren pharmazeutisch unbedenkliche Salze und Solvate, wie in Anspruch 1 definiert, ausgewählt aus:
N-(5-Chlor-2-{3-[3-(4-chlorphenoxy)pyrrolidin-1-yl]-2-hydroxypropoxy]phenyl)isobuttersäureamid,
N-(2-{3-[3-(4-Chlorphenoxy)pyrrolidin-1-yl]-2-hydroxypropoxy}phenyl]thiophen-2-carbonsäureamid,
N-[(2-{3-[3-(4-Chlorphenoxy)pyrrolidin-1-yl]-2-hydroxypropoxy}phenylcarbamoyl)methyl]benzamid,
N-(2-{3-[3-(4-Chlorphenoxy)pyrrolidin-1-yl]-2-hydroxypropoxy}phenyl)pyrazin-2-carbonsäureamid,
N-(2-{3-[3-(4-Chlorphenoxy)pyrrolidin-1-yl]-2-hydroxypropoxy}phenyl)cyclohexancarbonsäureamid,
N-(2-{3-[3-(4-Chlorphenoxy)pyrrolidin-1-yl]-2-hydroxypropoxy)phenyl)phthalsäureamidmethylester,
N-(2-{3-[3-(4-Chlorphenoxy)pyrrolidin-1-yl]-2-hydroxypropoxy}phenyl)-3-hydroxybuttersäureamid,
N-(2-{3-[3-(4-Chlorphenoxy)pyrrolidin-1-yl]-2-hydroxypropoxy}phenyl)-2-ureidoessigsäureamid,
4-Acetylamino-N-(2-{3-[3-(4-Chlorphenoxy)-pyrrolidin-1-yl]-2-hydroxypropoxy}phenyl)buttersäureamid,
N-(2-{3-[3-(4-Chlorphenoxy)pyrrolidin-1-yl]-2-hydroxypropoxy}phenyl)-1-acetylpiperidin-4-carbonsäureamid,
N-(2-{3-[3-(4-Chlorphenoxy)pyrrolidin-1-yl]-2-hydroxypropoxy}phenyl)-3-methoxybenzamid,
2-Acetylamino-N-(2-{3-[3-(4-chlorphenoxy)-pyrrolidin-1-yl]-2-hydroxypropoxy}phenyl)-3-methylbuttersäureamid,
2-Acetylamino-N-(2-{3-[3-(4-chlorphenoxy)-pyrrolidin-1-yl]-2-hydroxypropoxy}phenyl)-3-hydroxybuttersäureamid,
N-(2-{3-[3-(4-Chlorphenoxy)pyrrolidin-1-yl]-2-hydroxypropoxy}phenyl)adamantan-1-carbonsäureamid,
2-Acetylamino-N-(2-{3-[3-(4-chlorphenoxy)-pyrrolidin-1-yl]-2-hydroxypropoxy}phenyl)-3-phenylpropionsäureamid,
N-(2-{3-[3-(4-Chlorphenoxy)pyrrolidin-1-yl]-2-hydroxypropoxy}phenyl)-2-methoxybenzamid,
N-(2-{3-[3-(4-Chlorphenoxy)pyrrolidin-1-yl]-2-hydroxypropoxy}phenyl)-5-methylthiophen-2-carbonsäureamid,
N-(2-{3-[3-(4-Chlorphenoxy)pyrrolidin-1-yl]-2-hydroxypropoxy}phenyl)-1-acetylpyrrolidin-2-carbonsäureamid,
N-(2-{3-[3-(4-Chlorphenoxy)pyrrolidin-1-yl]-2-hydroxypropoxy}phenyl)-1,5-dimethyl-1H-pyrazol-3-carbonsäureamid,
N-(2-{3-[3-(4-Chlorphenoxy)pyrrolidin-1-yl]-2-hydroxypropoxy}phenyl)-5-oxo-pyrrolidin-2-carbonsäureamid,
N-(2-{3-[3-(4-Chlorghenoxy)pyrrolidin-1-yl]-2-hydroxypropoxy}phenyl)-1H-indol-6-carbonsäureamid,
N-(2-{3-[3-(4-Chlorphenoxy)pyrrolidin-1-yl]-2-hydroxypropoxy}phenyl)cyclobutancarbonsäureamid,
N-(2-{3-[3-(4-Chlorphenoxy)pyrrolidin-1-yl]-2-hydroxypropoxy)phenyl)propionsäureamid,
N-(2-{3-[3-(4-Chlorphenoxy)pyrrolidin-1-yl]-2-hydroxypropoxy}phenyl)pentansäureamid,
N-(2-{3-[3-(4-Chlorphenoxy)pyrrolidin-1-yl]-2-hydroxypropoxy}phenyl)pent-4-ensäureamid,
N-(2-{3-[3-(4-Chlorphenoxy)pyrrolidin-1-yl]-2-hydroxypropoxy}phenyl)cyclopentancarbonsäureamid,
N-(2-{3-[3-(4-Chlorphenoxy)pyrrolidin-1-yl]-2-hydroxypropoxy}phenyl)cyclopropancarbonsäureamid,
N-(2-{3-[3-(4-Chlorphenoxy)pyrrolidin-1-yl]-2-hydroxypropoxy}phenyl)isobuttersäureamid,
N-(2-{3-[3-(4-Chlorphenoxy)pyrrolidin-1-yl]-2-hydroxypropoxy)phenyl)-2-methylsufanylessigsäureamid,
2-Acetylamino-N-(2-{3-[3-(4-chlorphenoxy)-pyrrolidin-1-yl]-2-hydroxypropoxy}phenyl)propionsäureamid,
N-(2-{3-[3-(4-Chlorphenoxy)pyrrolidin-1-yl]-2-hydroxypropoxy}phenyl)buttersäureamid,
N-(2-{3-[3-(4-Chlorphenoxy)pyrrolidin-1-yl]-2-hydroxypropoxy}phenyl)-3-methylbuttersäureamid,
N-(2-{3-[3-(4-Chlorphenoxy)pyrrolidin-1-yl]-2-hydroxypropoxy}phenyl)-2-methoxyessigsäureamid,
N-(2-{3-[3-(4-Chlorphenoxy)pyrrolidin-1-yl]-2-hydroxypropoxy}phenyl)-2,2-dimethylpropionsäureamid,
N-(2-{3-[3-(4-Chlorphenoxy)pyrrolidin-1-yl]-2-hydroxypropoxy}phenyl)-5-oxohexansäureamid,
N-(2-{3-[3-(4-Chlorphenoxy)pyrrolidin-1-yl]-2-hydroxypropoxy}phenyl)hexansäureamid,
2-Chlor-N-(2-{3-[3-(4-Chlorphenoxy)pyrrolidin-1-yl]-2-hydroxypropoxy}phenyl)benzamid,
3-Chlor-N-(2-{3-[3-(4-Chlorphenoxy)pyrrolidin-1-yl]-2-hydroxypropoxy}phenyl)benzamid,
(4R)-N-(2-{3-[3-(4-Chlorphenoxy)-1-pyrrolidin]-2-hydroxypropoxy}phenyl)-1,3-thiazolidin-4-carbonsäureamid-ditrifluoracetat,
N-(2-{3-[4-(3,4-Dichlorphenoxy)piperidin-1-yl]-2-hydroxypropoxy}phenyl)thiophen-2-carbonsäureamid,
N-(2-{3-[4-(3,4-Dichlorphenoxy)piperidin-1-yl]-2-hydroxypropoxy)phenyl)benzamid,
N-(2-{3-[4-(3,4-Dichlorphenoxy)piperidin-1-yl]-2-hydroxypropoxy}phenyl)nicotinsäureamid,
N-(2-{3-[4-(3,4-Dichlorphenoxy)piperidin-1-yl]-2-hydroxypropoxy}phenyl)pyridin-2-carbonsäureamid,
N-(2-{3-[4-(3,4-Dichlorphenoxy)piperidin-1-yl]-2-hydroxypropoxy}phenyl)isonicotinsäureamid,
N-(2-{3-[4-(3,4-Dichlorphenoxy)piperidin-1-yl]-2-hydroxypropoxy}phenyl)cyclohexancarbonsäureamid,
N-(2-{3-[4-(3,4-Dichlorphenoxy)piperidin-1-yl]-2-hydroxypropoxy}phenyl)-3-hydroxybuttersäureamid,
N-(2-{3-[4-(3,4-Dichlorphenoxy)piperidin-1-yl]-2-hydroxypropoxy}phenyl)-5-methylthiophen-2-carbonsäureamid,
N-(2-{3-[4-(3,4-Dichlorphenoxy)piperidin-1-yl]-2-hydroxypropoxy}phenyl)cyclobutancarbonsäureamid,
N-(2-{3-[4-(3,4-Dichlorphenoxy)piperidin-1-yl]-2-hydroxypropoxy}phenyl)propionsäureamid,
N-(2-{3-[4-(3,4-Dichlorphenoxy)piperidin-1-yl]-2-hydroxypropoxy}phenyl)pentansäureamid,
N-(2-{3-[4-(3,4-Dichlorphenoxy)piperidin-1-yl]-2-hydroxypropoxy}phenyl)pent-4-ensäureamid,
N-(2-{3-[4-(3,4-Dichlorphenoxy)piperidin-1-yl]-2-hydroxypropoxy}phenyl)cyclopentancarbonsäureamid,
N-(2-{3-[4-(3,4-Dichlorphenoxy)piperidin-1-yl]-2-hydroxypropoxy}phenyl)-3-methylbuttersäureamid,
N-(2-{3-[3-(4-Chlorphenoxy)-1-pyrrolidin]-2-hydroxypropoxy}phenyl)-2,2,2-trifluoressigsäurehydrochlorid,
4-(2-{3-[4-(3,4-Dichlorphenoxy)piperidin-1-yl]-2-hydroxypropoxy}phenylcarbamoyl)-3-methylbuttersäure,
N-(2-{3-[4-(3,4-Dichlorphenoxy)piperidin-1-yl]-2-hydroxypropoxy}phenyl)bernsteinsäureamid,
N-(2-{3-[3-(4-Chlorphenoxy)pyrrolidin-1-yl]-2-hydroxypropoxy}-4-methylphenyl)furan-2-carbonsäureamid,
N-(2-{3-[3-(4-Chlorphenoxy)pyrrolidin-1-yl]-2-hydroxypropoxy)-4-methylphenyl)-1H-pyrrol-2-carbonsäureamid,
N-(2-{3-[3-(4-Chlorphenoxy)pyrrolidin-1-yl]-2-hydroxypropoxy)-4-methylphenyl)thiophen-2-carbonsäureamid,
N-(2-{3-[3-(4-Chlorphenoxy)pyrrolidin-1-yl]-2-hydroxypropoxy}-4-methylphenyl)cyclopentancarbonsäureamid,
N-(2-{3-[3-(4-Chlorphenoxy)pyrrolidin-1-yl]-2-hydroxypropoxy}-4-methylphenyl)-5-methylthiophen-2-carbonsäureamid,
N-(2-{3-[3-(4-Chlorphenoxy)pyrrolidin-1-yl]-2-hydroxypropoxy}-4-methylphenyl)-3-chlorthiophen-2-carbonsäureamid,
N-(2-{3-[3-(4-Chlorphenoxy)pyrrolidin-1-yl]-2-hydroxypropoxy}-4-methylphenyl)-5-methylisoxazol-4-carbonsäureamid,
N-(2-{3-[3-(4-Chlorphenoxy)pyrrolidin-1-yl]-2-hydroxypropoxy}-4-methylphenyl)-[1,2,3]-thiadiazol-4-carbonsäureamid,
N-(2-{3-[3-(4-Chlorphenoxy)pyrrolidin-1-yl]-2-hydroxypropoxy}-4-methylphenyl)-3-methylfuran-2-carbonsäureamid,
N-(2-{3-[3-(4-Chlorphenoxy)pyrrolidin-1-yl]-2-hydroxypropoxy}-4-methylphenyl)cyclopent-1-encarbonsäureamid,
N-(2-{3-[3-(4-Chlorphenoxy)pyrrolidin-1-yl]-2-hydroxypropoxy}-4-methylphenyl)-2-methylfuran-3-carbonsäuramid,
N-(2-{3-[3-(4-Chlorphenoxy)pyrrolidin-1-yl]-2-hydroxypropoxy}-4-methylphenyl)-3-methylthiophen-2-carbonsäureamid,
N-(2-{3-[3-(4-Chlorphenoxy)pyrrolidin-1-yl]-2-hydroxypropoxy}-4-methylphenyl}-5-nitro-1H-pyrazol-3-carbonsäureamid,
N-(2-{3-[3-(4-Chlorphenoxy)pyrrolidin-1-yl]-2-hydroxypropoxy}-4-methylphenyl)thiophen-3-carbonsäureamid,
N-(2-{3-[3-(4-Chlorphenoxy)pyrrolidin-1-yl]-2-hydroxypropoxy}-4-methylphenyl)cyclobutancarbonsäureamid,
N-(2-{3-[3-(4-Fluorphenoxy)pyrrolidin-1-yl]-2-hydroxypropoxy}-4-methylphenyl)furan-2-carbonsäureamid,
N-(2-{3-[3-(4-Fluorphenoxy)pyrrolidin-1-yl]-2-hydroxypropoxy}-4-methylphenyl)-1H-pyrrol-2-carbonsäureamid,
N-(2-{3-[3-(4-Fluorphenoxy)pyrrolidin-1-yl]-2-hydroxypropoxy}-4-methylphenyl)thiophen-2-carbonsäureamid,
N-(2-{3-[3-(4-Fluorphenoxy)pyrrolidin-1-yl]-2-hydroxypropoxy}-4-methylphenyl)-3-chlorthiophen-2-carbonsäureamid,
N-(2-{3-[3-(4-Fluorphenoxy)pyrrolidin-1-yl]-2-hydroxypropoxy}-4-methylphenyl)-5-methylisoxazol-4-carbonsäureamid,
N-(2-{3-[3-(4-Fluorphenoxy)pyrrolidin-1-yl]-2-hydroxypropoxy}-4-methylphenyl)-3-methylfuran-2-carbonsäureamid,
N-(2-{3-[3-(4-Fluorphenoxy)pyrrolidin-1-yl]-2-hydroxypropoxy}-4-methylphenyl)cyclopent-1-encarbonsäureamid,
N-(2-{3-[3-(4-Fluorphenoxy)pyrrolidin-1-yl]-2-hydroxypropoxy}-4-methylphenyl)-2-methylfuran-3-carbonsäureamid,
N-(2-{3-[3-(4-Fluorphenoxy)pyrrolidin-1-yl]-2-hydroxypropoxy}-4-methylphenyl)-3-methylthiophen-2-carbonsäureamid,
N-(2-{3-[3-(4-Fluorphenoxy)pyrrolidin-1-yl]-2-hydroxypropoxy}-4-methylphenyl)-5-chlorthiophen-2-carbonsäureamid,
N-(2-{3-[3-(4-Fluorphenoxy)pyrrolidin-1-yl]-2-hydroxypropoxy}-4-methylphenyl)thiophen-3-carbonsäureamid,
N-(2-{3-[3-(4-Fluorphenoxy)pyrrolidin-1-yl]-2-hydroxypropoxy}-4-methylphenyl)-2,5-dimethylfuran-3-carbonsäureamid,
N-(2-{3-[3-(4-Fluorphenoxy)pyrrolidin-1-yl]-2-hydroxypropoxy}-4-methylphenyl)cyclobutancarbonsäureamid,
N-(2-{3-[3-(4-Fluorphenoxy)pyrrolidin-1-yl]-2-hydroxypropoxy}-4-methylphenyl)furan-3-carbonsäureamid,
N-{2-[(3-{3-[(4-Fluorphenyl)oxy]-1-pyrrolidinyl}-2-hydroxypropyl)oxy]-4-methylphenyl}-1H-pyrrol-2-carbonsäureamid,
N-{2-[(3-{3-[(4-Chlorphenyl)oxy]-1-pyrrolidinyl}-2-hydroxypropyl)oxy]-4-methylphenyl}-3-thiophencarbonsäureamid,
N-{2-[(3-{3-[(4-Chlorphenyl)oxy]-1-pyrrolidinyl}-2-hydroxy-2-methylpropyl)oxy]phenyl}-2-thiophencarbonsäureamid, Verbindung mit Trifluoressigsäure,
N-{2-[(3-{3-[(4-Fluorphenyl)oxy]-1-pyrrolidinyl}-2-hydroxypropyl)oxy]-4-methylphenyl}-2-thiophencarbonsäureamid,
N-{2-[(3-{3-[(4-Chlorphenyl)oxy]-1-pyrrolidinyl}-2-hydroxypropyl)oxy]phenyl}-2-furancarbonsäureamid,
N-{2-[(3-{3-[(4-Chlorphenyl)oxy]-1-pyrrolidinyl}-2-hydroxypropyl)oxy]phenyl}-1-pyrrol-2-carbonsäureamid,
N-{2-[(3-{3-[(4-Chlarphenyl)oxy]-1-pyrrolidinyl}-2-hydroxypropyl)oxy]-4-methylphenyl}-1H-pyrrol-3-carbonsäureamid,
N-{2-[(3-{3-[(4-Fluorphenyl)oxy]-1-pyrrolidinyl}-2-hydroxypropyl)oxy]-4-methylphenyl}-2-furancarbonsäureamid,
N-{2-[(3-{3-[(4-Chlorphenyl)oxy]-1-pyrrolidinyl}-2-hydroxy-2-methylpropyl)oxy]phenyl}cyclopentancarbonsäureamid, Verbindung mit Trifluoressigsäure,
N-(2-{3-[3-(4-Fluorphenoxy)pyrrolidin-1-yl]-2-hydroxy-2-methylpropoxy}phenyl}benzamid,
N-(2-{3-[3-(4-Cyanophenoxy)pyrrolidin-1-yl]-2-hydroxy-2-methylpropoxy)phenyl}benzamid,
N-(2-{3-[4-(3,4-Dichlorphenoxy)piperidin-1-yl]-2-hydroxy-2-methylpropoxy}phenyl}benzamid,
N-(2-{3-[3-(4-Chlorphenoxy)pyrrolidin-1-yl]-2-hydroxy-2-methylpropoxy}phenyl}benzamid und
N-(2-{3-[4-(3,4-Dichlorphenylamino)piperidin-1-yl]-2-hydroxy-2-methylpropoxy}phenyl}benzamid.

9. Verfahren zur Herstellung einer wie in Anspruch 1 definierten Verbindung der Formel (I), bei dem man eine Verbindung der allgemeinen Formel 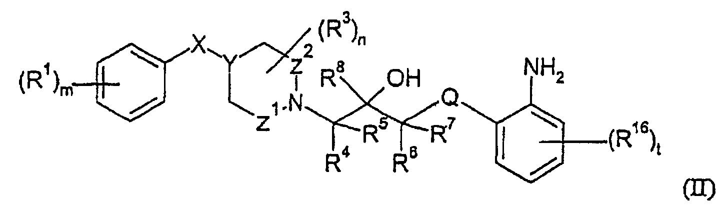 oder ein Salz davon, wobei m, n, t, R¹, R³, R⁴, R⁵, R⁶, R⁷, R⁸, R¹⁶, Q, Z¹ und Z² wie in Formel (I) definiert sind, mit einer Verbindung der allgemeinen Formel
R¹⁵ - CO₂H (III)
oder einem chemischen Äquivalent davon, wobei R¹⁵ wie in Formel (I) definiert ist, umsetzt;
und anschließend gegebenenfalls ein pharmazeutisch unbedenkliches Salz oder Solvat der so erhaltenen Verbindung der Formel (I) bildet.

10. Pharmazeutische Zusammensetzung, enthaltend eine Verbindung der Formel (I) oder ein pharmazeutisch unbedenkliches Salz oder Solvat davon nach einem der Ansprüche 1 bis 8 zusammen mit einem pharmazeutisch unbedenklichen Adjuvans, Verdünnungsmittel oder Träger.

11. Verfahren zur Herstellung einer pharmazeutischen Zusammensetzung nach Anspruch 10, bei dem man eine Verbindung der Formel (I) oder ein pharmazeutisch unbedenkliches Salz oder Solvat davon nach einem der Ansprüche 1 bis 8 mit einem pharmazeutisch unbedenklichen Adjuvans, Verdünnungsmittel oder Träger mischt.

12. Verbindungen der Formel (I) und deren pharmazeutisch unbedenkliche Salze und Solvate nach einem der Ansprüche 1 bis 8 zur Verwendung in der Therapie.

13. Verwendung einer Verbindung der Formel (I) oder eines pharmazeutisch unbedenklichen Salzes oder Solvates davon nach einem der Ansprüche 1 bis 8 bei der Herstellung eines Medikaments zur Verwendung in der Therapie.

14. Verwendung einer Verbindung der Formel (I) oder eines pharmazeutisch unbedenklichen Salzes oder Solvates davon nach einem der Ansprüche 1 bis 8 bei der Herstellung eines Medikaments zur Behandlung von menschlichen Erkrankungen oder Leiden, bei denen die Modulierung der Chemokinrezeptoraktivität von Nutzen ist.

15. Verwendung einer Verbindung der Formel (I) oder eines pharmazeutisch unbedenklichen Salzes oder Solvates davon nach einem der Ansprüche 1 bis 8 bei der Herstellung eines Medikaments zur Verwendung bei der Behandlung von rheumatoider Arthritis.

16. Verwendung einer Verbindung der Formel (I) oder eines pharmazeutisch unbedenklichen Salzes oder Solvates davon nach einem der Ansprüche 1 bis 8 bei der Herstellung eines Medikaments zur Verwendung bei der Behandlung von chronischobstruktiver Atemwegserkrankung.

17. Verwendung einer Verbindung der Formel (I) oder eines pharmazeutisch unbedenklichen Salzes oder Solvates davon nach einem der Ansprüche 1 bis 8 bei der Herstellung eines Medikaments zur Verwendung bei der Behandlung von Asthma.

18. Verwendung einer Verbindung der Formel (I) oder eines pharmazeutisch unbedenklichen Salzes oder Solvates davon nach einem der Ansprüche 1 bis 8 bei der Herstellung eines Medikaments zur Verwendung bei der Behandlung von multipler Sklerose.

## Revendications

1. Composé de formule générale 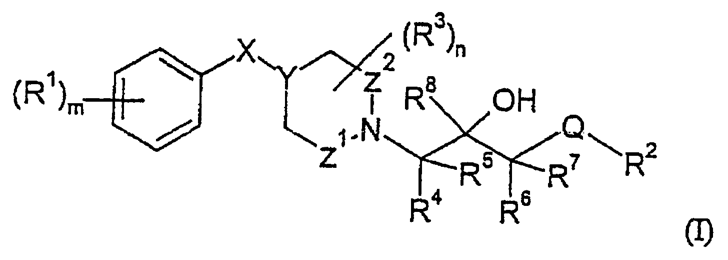 dans laquelle :
m vaut 0, 1, 2 ou 3 ;
chaque R¹ représente indépendamment un halogène, un cyano, un nitro, un carboxy, un hydroxy, un cycloalkyle en C₃-C₆, un alcoxy en C₁-C₆, un C₁-C₆-alcoxycarbonyle, un halogénoalkyle en C₁-C₆, un halogénoalcoxy en C₁-C₆, -NR⁹R¹⁰, un C₃-C₆-cycloalkylamino, un C₁-C₆-alkylthio, un C₁-C₆-alkylcarbonyle, un C₁-C₆-alkylcarbonylamino, un sulfonamido, un C₁-C₆-alkylsulfonyle, -C(O)NR¹¹R¹², -NR¹³C(O)-(NH)ₚR¹⁴, un phényle ou un alkyle en C₁-C₆ éventuellement substitué par un carboxy ou un C₁-C₆-alcoxycarbonyle ;
p vaut 0 ou 1 ;
X représente un atome d'oxygène ou un groupe CH₂, OCH₂, CH₂O, CH₂NH, NH, carbonyle ou sulfonyle et Y représente un atome d'azote ou un groupe CH ou C(OH), à condition que lorsque X représente un atome d'oxygène ou un groupe CH₂O, CH₂NH ou NH, alors Y représente un groupe CH ;
Z¹ représente une liaison ou un groupe (CH₂)_{q} dans lequel q vaut 1 ou 2 ;
Z² représente une liaison ou un groupe CH₂, à condition que Z¹ et Z² ne représentent pas simultanément tous les deux une liaison ;
Q représente un atome d'oxygène ou de soufre ou un groupe CH₂ ou NH ;
R² représente un groupe 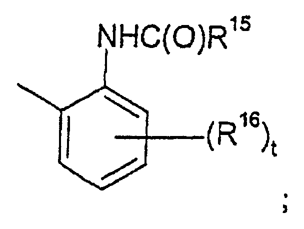
n vaut 0, 1 ou 2 ;
chaque R³ représente indépendamment un alkyle en C₁-C₆, un C₁-C₆-alcoxycarbonyle, -CH₂OH ou un groupe carboxy ;
R⁴, R⁵, R⁶ et R⁷ représentent chacun indépendamment un atome d'hydrogène ou un groupe alkyle en C₁-C₆, ou R⁴, R⁵, R⁶ et R⁷ représentent conjointement une chaîne alkylène en C₁-C₄ reliant les deux atomes de carbone auxquels ils sont fixés pour former un carbocycle saturé à 4 - 7 chaînons, ou R⁵, R⁶ et R⁷ représentent chacun un atome d'hydrogène et R⁴ et R⁸, conjointement avec les atomes de carbone auxquels ils sont fixés, forment un carbocycle saturé à 5 - 6 chaînons ;
R⁸ représente un atome d'hydrogène, un groupe alkyle en C₁-C₆ ou est lié à R⁴ tel que défini ci-dessus ;
R⁹ et R¹⁰ représentent chacun indépendamment un atome d'hydrogène ou un groupe alkyle en C₁-C₆, ou R⁹ et R¹⁰, conjointement avec l'atome d'azote auquel ils sont fixés, forment un hétérocycle saturé à 4 - 7 chaînons ;
R¹¹ et R¹² représentent chacun indépendamment un atome d'hydrogène ou un groupe alkyle en C₁-C₆ éventuellement substitué par un C₁-C₆-alcoxycarbonyle ;
R¹³ représente un atome d'hydrogène ou un groupe alkyle en C₁-C₆ ;
R¹⁴ représente un atome d'hydrogène ou un groupe alkyle en C₁-C₆ éventuellement substitué par un carboxy, un alcoxy en C₁-C₆ ou un C₁-C₆-alcoxycarbonyle ;
R¹⁵ représente un groupe alkyle en C₂-C₆, alcényle en C₂-C₆, cycloalkyle en C₃-C₆, cycloalcényle en C₅-C₆, adamantyle, phényle ou un système de noyau hétérocyclique saturé ou insaturé, à 5 - 10 chaînons, renfermant au moins un hétéroatome choisi parmi un azote, un oxygène et un soufre, dans lequel chaque groupe peut être éventuellement substitué par un ou plusieurs substituants choisis indépendamment parmi un nitro, un hydroxy, un oxo, un halogène, un carboxy, un alkyle en C₁-C₆, un alcoxy en C₁-C₆, un C₁-C₆-alkylthio, un C₁-C₆-alkylcarbonyle, un C₁-C₆-alcoxycarbonyle, un phényle et -NHC(O)-R¹⁷, à condition que R¹⁵ ne représente pas un groupe 1-pyrrolidinyle non substitué, 1-pipéridinyle non substitué ou 1-hexaméthylène-iminyle non substitué ;
t vaut 0, 1, 2 ou 3 ;
chaque R¹⁶ représente indépendamment un halogène, un cyano, un nitro, un carboxy, un hydroxy, un cycloalkyle en C₃-C₆, un alcoxy en C₁-C₆, un C₁-C₆-alcoxycarbonyle, un halogénoalkyle en C₁-C₆, un halogénoalcoxy en C₁-C₆, -NR¹⁸R¹⁹, un C₃-C₆-cycloalkylamino, un C₁-C₆-alkylthio, un C₁-C₆-alkylcarbonyle, un C₁-C₆-alkylcarbonylamino, un sulfonamido (-SO₂NH₂), un C₁-C₆-alkylsulfonyle, -C(O)NR²⁰R²¹, -NR²²C(O) (NH)ᵥR²³, un phényle, ou un alkyle en C₁-C₆ éventuellement substitué par un carboxy ou un C₁-C₆-alcoxycarbonyle ;
R¹⁷ représente un groupe alkyle en C₁-C₆, amino ou phényle ;
R¹⁸ et R¹⁹ représentent chacun indépendamment un atome d'hydrogène ou un groupe alkyle en C₁-C₆, ou R¹⁸ et R¹⁹, conjointement avec l'atome d'azote auquel ils sont fixés, forment un hétérocycle saturé à 4 - 7 chaînons ;
R²⁰ et R²¹ représentent chacun indépendamment un atome d'hydrogène ou un groupe alkyle en C₁-C₆ éventuellement substitué par un C₁-C₆-alcoxycarbonyle ;
v vaut 0 ou 1 ;
R²² représente un atome d'hydrogène ou un groupe alkyle en C₁-C₆ ; et
R²³ représente un atome d'hydrogène, ou un groupe alkyle en C₁-C₆ éventuellement substitué par un carboxy, un alcoxy en C₁-C₆ ou un C₁-C₆-alcoxycarbonyle ;
ou un sel ou solvate pharmaceutiquement acceptable de celui-ci.

2. Composé selon la revendication 1, dans lequel X représente un atome d'oxygène ou un groupe CH₂ ou NH.

3. Composé selon la revendication 1, dans lequel Y représente un groupe CH.

4. Composé selon l'une quelconque des revendications 1 à 3, dans lequel Q représente un atome d'oxygène.

5. Composé selon l'une quelconque des revendications 1 à 4, dans lequel R¹⁵ représente un groupe alkyle en C₂-C₅, alcényle en C₂-C₄, cycloalkyle en C₃-C₆, cycloalcényle en C₅-C₆, adamantyle, phényle ou un système de noyau hétérocyclique saturé ou insaturé, à 5 - 10 chaînons, renfermant au moins un hétéroatome choisi parmi un azote, un oxygène et un soufre, dans lequel chaque groupe peut être éventuellement substitué par un, deux ou trois substituants choisis indépendamment parmi un hydroxy, un oxo, un halogène, un carboxy, un alkyle en C₁-C₆, un alcoxy en C₁-C₆, un C₁-C₆-alkylthio, un C₁-C₆-alkylcarbonyle, un C₁-C₆-alcoxycarbonyle, un phényle et -NHC(O)-R¹⁷.

6. Composé selon la revendication 5, dans lequel le système de noyau hétérocyclique saturé ou insaturé, à 5 - 10 chaînons, renfermant au moins un hétéroatome choisi parmi un azote, un oxygène et un soufre, est un pyrrolidinyle, un pipéridinyle, un pyrazolyle, un thiazolidinyle, un thiényle, un thiadiazolyle, un isoxazolyle, un pyrrolyle, un furanyle, un thiazolyle, un indolyle, un quinoléinyle, un benzimidazolyle, un triazolyle, un tétrazolyle ou un pyridinyle.

7. Composé selon l'une quelconque des revendications 1 à 6, dans lequel chaque R¹⁶ représente indépendamment un halogène, un cyano, un alcoxy en C₁-C₄, un C₁-C₄-alcoxycarbonyle, un halogénoalkyle en C₁-C₄, un C₁-C₄-alkylcarbonyle, un phényle ou un alkyle en C₁-C₄.

8. Composé de formule (I), ou sel ou solvate pharmaceutiquement acceptable de celui-ci, tel que défini dans la revendication 1, choisi parmi :
le N-(5-chloro-2-{3-[3-(4-chlorophénoxy)-pyrrolidin-1-yl]-2-hydroxypropoxy}phényl)-isobutyramide,
le (2-{3-[3-(4-chlorophénoxy)pyrrolidin-1-yl]-2-hydroxypropoxy}phényl)amide de l'acide thiophène-2-carboxylique,
le N-[(2-{3-[3-(4-chlorophénoxy)pyrrolidin-1-yl]-2-hydroxypropoxy}phénylcarbamoyl)méthyl]benzamide,
le (2-{3-[3-(4-chlorophénoxy)pyrrolidin-1-yl]-2-hydroxypropoxy}phényl)amide de l'acide pyrazine-2-carboxylique,
le (2-{3-[3-(4-chlorophénoxy)pyrrolidin-1-yl]-2-hydroxypropoxy}phényl)amide de l'acide cyclohexanecarboxylique,
l'ester méthylique de l'acide N-(2-{3-[3-(4-chlorophénoxy)pyrrolidin-1-yl]-2-hydroxypropoxy}phényl)phtalamique,
le N-(2-{3-[3-(4-chlorophénoxy)pyrrolidin-1-yl]-2-hydroxypropoxy}phényl)-3-hydroxybutyramide,
le N-(2-{3-[3-(4-chlorophénoxy)pyrrolidin-1-yl]-2-hydroxypropoxy}phényl)-2-uréidoacétamide,
le 4-acétylamino-N-(2-{3-[3-(4-chlorophénoxy)-pyrrolidin-1-yl]-2-hydroxypropoxy}phényl)-butyramide,
le (2-{3-[3-(4-chlorophénoxy)pyrrolidin-1-yl]-2-hydroxypropoxy}phényl)amide de l'acide 1-acétylpipéridine-4-carboxylique,
le N-(2-{3-[3-(4-chlorophénoxy)pyrrolidin-1-yl]-2-hydroxypropoxy}phényl)-3-méthoxybenzamide,
le 2-acétylamino-N-(2-{3-[3-(4-chlorophénoxy)-pyrrolidin-1-yl]-2-hydroxypropoxy}phényl)-3-méthylbutyramide,
le 2-acétylamino-N-(2-{3-[3-(4-chlorophénoxy)-pyrrolidin-1-yl]-2-hydroxypropoxy}phényl)-3-hydroxybutyramide,
le (2-{3-[3-(4-chlorophénoxy)pyrrolidin-1-yl]-2-hydroxypropoxy}phényl)amide de l'acide adamantane-1-carboxylique,
le 2-acétylamino-N-(2-{3-[3-(4-chlorophénoxy)-pyrrolidin-1-yl]-2-hydroxypropoxy}phényl)-3-phénylpropionamide,
le N-(2-{3-[3-(4-chlorophénoxy)pyrrolidin-1-yl]-2-hydroxypropoxy}phényl)-2-méthoxybenzamide,
le (2-{3-[3-(4-chlorophénoxy)pyrrolidin-1-yl]-2-hydroxypropoxy}phényl)amide de l'acide 5-méthylthiophène-2-carboxylique,
le (2-{3-[3-(4-chlorophénoxy)pyrrolidin-1-yl]-2-hydroxypropoxy}phényl)amide de l'acide 1-acétylpyrrolidine-2-carboxylique,
le (2-{3-[3-(4-chlorophénoxy)pyrrolidin-1-yl]-2-hydroxypropoxy}phényl)amide de l'acide 1,5-diméthyl-1H-pyrazole-3-carboxylique,
le (2-{3-[3-(4-chlorophénoxy)pyrrolidin-1-yl]-2-hydroxypropoxy}phényl)amide de l'acide 5-oxo-pyrrolidine-2-carboxylique,
le (2-{3-[3-(4-chlorophénoxy)pyrrolidin-1-yl]-2-hydroxypropoxy}phényl)amide de l'acide 1H-indole-6-carboxylique,
le (2-{3-[3-(4-chlorophénoxy)pyrrolidin-1-yl]-2-hydroxypropoxy}phényl)amide de l'acide cyclobutanecarboxylique,
le N-(2-{3-[3-(4-chlorophénoxy)pyrrolidin-1-yl]-2-hydroxypropoxy}phényl)propionamide,
le (2-{3-[3-(4-chlorophénoxy)pyrrolidin-1-yl]-2-hydroxypropoxy}phényl)amide de l'acide pentanoïque,
le (2-{3-[3-(4-chlorophénoxy)pyrrolidin-1-yl]-2-hydroxypropoxy}phényl)amide de l'acide pent-4-énoïque,
le (2-{3-[3-(4-chlorophénoxy)pyrrolidin-1-yl]-2-hydroxypropoxy}phényl)amide de l'acide cyclopentanecarboxylique,
le (2-{3-[3-(4-chlorophénoxy)pyrrolidin-1-yl]-2-hydroxypropoxy}phényl)amide de l'acide cyclopropanecarboxylique,
le N-(2-{3-[3-(4-chlorophénoxy)pyrrolidin-1-yl]-2-hydroxypropoxy}phényl)isobutyramide,
le N-(2-{3-[3-(4-chlorophénoxy)pyrrolidin-1-yl]-2-hydroxypropoxy}phényl)-2-méthylsulfanylacétamide,
le 2-acétylamino-N-(2-{3-[3-(4-chlorophénoxy)-pyrrolidin-1-yl]-2-hydroxypropoxy}phényl)propionamide,
le N-(2-{3-[3-(4-chlorophénoxy)pyrrolidin-1-yl]-2-hydroxypropoxy}phényl)butyramide,
le N-(2-{3-[3-(4-chlorophénoxy)pyrrolidin-1-yl]-2-hydroxypropoxy}phényl)-3-méthylbutyramide,
le N-(2-{3-[3-(4-chlorophénoxy)pyrrolidin-1-yl]-2-hydroxypropoxy}phényl)-2-méthoxyacétamide,
le N-(2-{3-[3-(4-chlorophénoxy)pyrrolidin-1-yl]-2-hydroxypropoxy}phényl)-2,2-diméthylpropionamide,
le (2-{3-[3-(4-chlorophénoxy)pyrrolidin-1-yl]-2-hydroxypropoxy}phényl)amide de l'acide 5-oxohexanoïque,
le (2-{3-[3-(4-chlorophénoxy)pyrrolidin-1-yl]-2-hydroxypropoxy}phényl)amide de l'acide hexanoïque,
le 2-chloro-N-(2-{3-[3-(4-chlorophénoxy)-pyrrolidin-1-yl]-2-hydroxypropoxy}phényl)-benzamide,
le 3-chloro-N-(2-{3-[3-(4-chlorophénoxy)-pyrrolidin-1-yl]-2-hydroxypropoxy}phényl)-benzamide,
le ditrifluoroacétate de (4*R*)-N-(2-{3-[3-(4-chlorophénoxy)-1-pyrrolidinyl]-2-hydroxypropoxy)-phényl)-1,3-thiazolidine-4-carboxamide,
le (2-{3-[9-(3,4-dichlorophénoxy)pipéridin-1-yl]-2-hydroxypropoxy}phényl)amide de l'acide thiophène-2-carboxylique,
le N-(2-{3-[4-(3,4-dichlorophénoxy)pipéridin-1-yl]-2-hydroxypropoxy}phényl)benzamide,
le N-(2-{3-[4-(3,4-dichlorophénoxy)pipéridin-1-yl]-2-hydroxypropoxy}phényl)nicotinamide,
le (2-{3-[4-(3,4-dichlorophénoxy)pipéridin-1-yl]-2-hydroxypropoxy}phényl)amide de l'acide pyridine-2-carboxylique,
le N-(2-{3-[4-(3,4-dichlorophénoxy)pipéridin-1-yl]-2-hydroxypropoxy}phényl)isonicotinamide,
le (2-{3-[4-(3,4-dichlorophénoxy)pipéridin-1-yl]-2-hydroxypropoxy}phényl)amide de l'acide cyclohexanecarboxylique,
le N-(2-{3-[4-(3,4-dichlorophénoxy)pipéridin-1-yl]-2-hydroxypropoxy}phényl)-3-hydroxybutyramide,
le (2-{3-[4-(3,4-dichlorophénoxy)pipéridin-1-yl]-2-hydroxypropoxy}phényl)amide de l'acide 5-méthylthiophène-2-carboxylique,
le (2-{3-[4-(3,4-dichlorophénoxy)pipéridin-1-yl]-2-hydroxypropoxy}phényl)amide de l'acide cyclobutanecarboxylique,
le N-(2-{3-[4-(3,4-dichlorophénoxy)pipéridin-1-yl]-2-hydroxypropoxy}phényl)propionamide,
le (2-{3-[4-(3,4-dichlorophénoxy)pipéridin-1-yl]-2-hydroxypropoxy}phényl)amide de l'acide pentanoïque,
le (2-{3-[4-(3,4-dichlorophénoxy)pipéridin-1-yl]-2-hydroxypropoxy}phényl)amide de l'acide pent-4-énoïque,
le (2-{3-[4-(3,4-dichlorophénoxy)pipéridin-1-yl]-2-hydroxypropoxy}phényl)amide de l'acide cyclopentanecarboxylique,
le N-(2-{3-[4-(3,4-dichlorophénoxy)pipéridin-1-yl]-2-hydroxypropoxy}phényl)-3-méthylbutyramide,
le chlorhydrate de N-(2-{3-[3-(4-chlorophénoxy)-1-pyrrolidinyl]-2-hydroxypropoxy}phényl)-2,2,2-trifluoroacétamide,
l'acide 4-(2-{3-[4-(3,4-dichlorophénoxy)pipéridin-1-yl]-2-hydroxypropoxy}phénylcarbamoyl)-3-méthylbutyrique,
l'acide N-(2-{3-[4-(3,4-dichlorophénoxy)pipéridin-1-yl]-2-hydroxypropoxy}phényl)succinamique,
le (2-{3-[3-(4-chlorophénoxy)pyrrolidin-1-yl]-2-hydroxypropoxy}-4-méthylphényl}amide de l'acide furane-2-carboxylique,
le (2-{3-[3-(4-chlorophénoxy)pyrrolidin-1-yl]-2-hydroxypropoxy}-4-méthylphényl)amide de l'acide 1H-pyrrole-2-carboxylique,
le (2-{3-[3-(4-chlorophénoxy)pyrrolidin-1-yl]-2-hydroxypropoxy}-4-méthylphényl)amide de l'acide thiophène-2-carboxylique,
le (2-{3-[3-(4-chlorophénoxy)pyrrolidin-1-yl]-2-hydroxypropoxy}-4-méthylphényl)amide de l'acide cyclopentanecarboxylique,
le (2-{3-[3-(4-chlorophénoxy)pyrrolidin-1-yl]-2-hydroxypropoxy)-4-méthylphényl)amide de l'acide 5-méthylthiophène-2-carboxylique,
le (2-{3-[3-(4-chlorophénoxy)pyrrolidin-1-yl]-2-hydroxypropoxy}-4-méthylphényl)amide de l'acide 3-chlorothiophène-2-carboxylique,
le (2-{3-[3-(4-chlorophénoxy)pyrrolidin-1-yl]-2-hydroxypropoxy}-4-méthylphényl)amide de l'acide 5-méthylisoxazole-4-carboxylique,
le (2-{3-[3-(4-chlorophénoxy)pyrrolidin-1-yl]-2-hydroxypropoxy}-4-méthylphényl)amide de l'acide [1,2,3]thiadiazole-4-carboxylique,
le (2-{3-[3-(4-chlorophénoxy)pyrrolidin-1-yl]-2-hydroxypropoxy}-4-méthylphényl)amide de l'acide 3-méthylfurane-2-carboxylique,
le (2-{3-[3-(4-chlorophénoxy)pyrrolidin-1-yl]-2-hydroxypropoxy}-4-méthylphényl)amide de l'acide cyclopent-1-ènecarboxylique,
le (2-{3-[3-(4-chlorophénoxy)pyrrolidin-1-yl]-2-hydroxypropoxy}-4-méthylphényl)amide de l'acide 2-méthylfurane-3-carboxylique,
le (2-{3-[3-(4-chlorophénoxy)pyrrolidin-1-yl]-2-hydroxypropoxy}-4-méthylphényl)amide de l'acide 3-méthylthiophène-2-carboxylique,
le (2-{3-[3-(4-chlorophénoxy)pyrrolidin-1-yl]-2-hydroxypropoxy}-4-méthylphényl)amide de l'acide 5-nitro-1H-pyrazole-3-carboxylique,
le (2-{3-[3-(4-chlorophénoxy)pyrrolidin-1-yl]-2-hydroxypropoxy}-4-méthylphényl)amide de thiophène-3-carboxylique,
le (2-{3-[3-(4-chlorophénoxy)pyrrolidin-1-yl]-2-hydroxypropoxy}-4-méthylphényl)amide de l'acide cyclobutanecarboxylique,
le (2-{3-[3-(4-fluorophénoxy)pyrrolidin-1-yl]-2-hydroxypropoxy}-4-méthylphényl)amide de l'acide furane-2-carboxylique,
le (2-{3-[3-(4-fluorophénoxy)pyrrolidin-1-yl]-2-hydroxypropoxy}-4-méthylphényl)amide de l'acide 1H-pyrrole-2-carboxylique,
le (2-{3-[3-(4-fluorophénoxy)pyrrolidin-1-yl]-2-hydroxypropoxy}-4-méthylphényl)amide de l'acide thiophène-2-carboxylique,
le (2-{3-[3-(4-fluorophénoxy)pyrrolidin-1-yl]-2-hydroxypropoxy}-4-méthylphényl)amide de l'acide 3-chlorothiophène-2-carboxylique,
le (2-{3-[3-(4-fluorophénoxy)pyrrolidin-1-yl]-2-hydroxypropoxy)-4-méthylphényl)amide de l'acide 5-méthylisoxazole-4-carboxylique,
le (2-{3-[3-(4-fluorophénoxy)pyrrolidin-1-yl]-2-hydroxypropoxy}-4-méthylphényl)amide de l'acide 3-méthylfurane-2-carboxylique,
le (2-{3-[3-(4-fluorophénoxy)pyrrolidin-1-yl]-2-hydroxypropoxy}-4-méthylphényl)amide de l'acide cyclopent-1-ènecarboxylique,
le (2-{3-[3-(4-fluorophénoxy)pyrrolidin-1-yl]-2-hydroxypropoxy}-4-méthylphényl)amide de l'acide 2-méthylfurane-3-carboxylique,
le (2-{3-[3-(4-fluorophénoxy)pyrrolidin-1-yl]-2-hydroxypropoxy}-4-méthylphényl)amide de l'acide 3-méthylthiophène-2-carboxylique,
le (2-{3-[3-(4-fluorophénoxy)pyrrolidin-1-yl]-2-hydroxypropoxy}-4-méthylphényl)amide de l'acide 5-chlorothiophène-2-carboxylique,
le (2-{3-[3-(4-fluorophénoxy)pyrrolidin-1-yl]-2-hydroxypropoxy}-4-méthylphényl)amide de l'acide thiophène-3-carboxylique,
le 2-{3-[3-(4-fluorophénoxy)pyrrolidin-1-yl]-2-hydroxypropoxy}-4-méthylphényl)amide de l'acide 2,5-diméthylfurane-3-carboxylique,
le (2-{3-[3-(4-fluorophénoxy)pyrrolidin-1-yl]-2-hydroxypropoxy}-4-méthylphényl)amide de l'acide cyclobutanecarboxylique,
le (2-{3-[3-(4-fluorophénoxy)pyrrolidin-1-yl]-2-hydroxypropoxy}-4-méthylphényl)amide de l'acide furane-3-carboxylique,
le N-{2-[(3-{3-[(4-fluorophényl)oxy]-1-pyrrolidinyl}-2-hydroxypropyl)oxy]-4-méthylphényl}-1H-pyrrole-2-carboxamide,
le N-{2-[(3-{3-[(4-chlorophényl)oxy]-1-pyrrolidinyl}-2-hydroxypropyl)oxy]-4-méthylphényl}-3-thiophène-carboxamide,
le N-{2-[(3-{3-[(4-chlorophényl)oxy]-1-pyrrolidinyl}-2-hydroxy-2-méthylpropyl)oxy]phényl}-2-thiophène-carboxamide, composé avec l'acide trifluoroacétique,
le N-{2-[(3-{3-[(4-fluorophényl)oxy]-1-pyrrolidinyl}-2-hydroxypropyl)oxy]-4-méthylphényl}-2-thiophène-carboxamide,
le N-{2-[(3-{3-[(4-chlorophényl)oxy]-1-pyrrolidinyl}-2-hydroxypropyl)oxy]phényl}-2-furanecarboxamide,
le N-{2-[(3-{3-[(4-chlorophényl)oxy]-1-pyrrolidinyl}-2-hydroxypropyl)oxy]phényl}-1-pyrrole-2-carboxamide,
le N-{2-[(3-{3-[(4-chlorophényl)oxy]-1-pyrrolidinyl}-2-hydroxypropyl)oxy]-4-méthylphényl}-1H-pyrrole-3-carboxamide,
le N-{2-[(3-{3-[(4-fluorophényl)oxy]-1-pyrrolidinyl}-2-hydroxypropyl)oxy]-4-méthylphényl}-2-furanecarboxamide,
le N-{2-[(3-{3-[(4-chlorophényl)oxy]-1-pyrrolidinyl}-2-hydroxy-2-méthylpropyl)oxy]phényl}cyclopentanecarboxamide, composé avec l'acide trifluoroacétique,
le N-(2-{3-[3-(4-fluorophénoxy)pyrrolidin-1-yl]-2-hydroxy-2-méthylpropoxy}phényl)benzamide,
le N-(2-{3-[3-(4-cyanophénoxy)pyrrolidin-1-yl]-2-hydroxy-2-méthylpropoxy}phényl)benzamide,
le N-(2-{3-[4-(3,4-dichlorophénoxy)pipéridin-1-yl]-2-hydroxy-2-méthylpropoxy}phényl)benzamide,
le N-(2-{3-[3-(4-chlorophénoxy)pyrrolidin-1-yl]-2-hydroxy-2-méthylpropoxy}phényl)benzamide, et
le N-(2-{3-[4-(3,4-dichlorophénylamino)pipéridin-1-yl]-2-hydroxy-2-méthylpropoxy}phényl)benzamide.

9. Procédé de préparation d'un composé de formule (I) tel que défini dans la revendication 1, comprenant la réaction d'un composé de formule générale 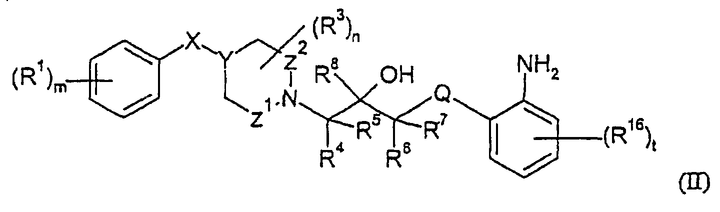 ou d'un sel de celui-ci, dans laquelle m, n, t, R¹, R³, R⁴, R⁵, R⁶, R⁷, R⁸, R¹⁶, Q, Z¹ et Z² sont tels que définis dans la formule (I), avec un composé de formule générale
R¹⁵ - CO₂H (III)
ou un dérivé chimiquement équivalent de celui-ci, dans laquelle R¹⁵ est tel que défini dans la formule (I) ;
et ensuite, éventuellement la formation d'un sel ou solvate pharmaceutiquement acceptable du composé de formule (I) obtenu.

10. Composition pharmaceutique comprenant un composé de formule (I) ou un sel ou solvate pharmaceutiquement acceptable de celui-ci, selon l'une quelconque des revendications 1 à 8, en association avec un adjuvant, un diluant ou un support pharmaceutiquement acceptable.

11. Procédé de préparation d'une composition pharmaceutique selon la revendication 10, comprenant la mixtion d'un composé de formule (I), ou d'un sel ou solvate pharmaceutiquement acceptable de celui-ci, selon l'une quelconque des revendications 1 à 8, avec un adjuvant, un diluant ou un support pharmaceutiquement acceptable.

12. Composé de formule (I), ou sel ou solvate pharmaceutiquement acceptable de celui-ci, selon l'une quelconque des revendications 1 à 8, destiné à être utilisé en thérapie.

13. Utilisation d'un composé de formule (I), ou d'un sel ou solvate pharmaceutiquement acceptable de celui-ci, selon l'une quelconque des revendications 1 à 8 pour la fabrication d'un médicament destiné à être utilisé en thérapie.

14. Utilisation d'un composé de formule (I), ou d'un sel ou solvate pharmaceutiquement acceptable de celui-ci, selon l'une quelconque des revendications 1 à 8 pour la fabrication d'un médicament pour le traitement de maladies ou d'états pathologiques humains pour lesquels une modulation de l'activité de récepteurs de chimiokines est bénéfique.

15. Utilisation d'un composé de formule (I), ou d'un sel ou solvate pharmaceutiquement acceptable de celui-ci, selon l'une quelconque des revendications 1 à 8 pour la fabrication d'un médicament destiné à être utilisé pour le traitement de la polyarthrite rhumatoïde.

16. Utilisation d'un composé de formule (I), ou d'un sel ou solvate pharmaceutiquement acceptable de celui-ci, selon l'une quelconque des revendications 1 à 8 pour la fabrication d'un médicament destiné à être utilisé pour le traitement d'une maladie pulmonaire obstructive chronique.

17. Utilisation d'un composé de formule (I), ou d'un sel ou solvate pharmaceutiquement acceptable de celui-ci, selon l'une quelconque des revendications 1 à 8 pour la fabrication d'un médicament destiné à être utilisé pour le traitement de l'asthme.

18. Utilisation d'un composé de formule (I), ou d'un sel ou solvate pharmaceutiquement acceptable de celui-ci, selon l'une quelconque des revendications 1 à 8 pour la fabrication d'un médicament destiné à être utilisé pour le traitement de la sclérose en plaques.
